# EUROPEAN PATENT SPECIFICATION

(11) **EP 3 843 825 B1**
(45) Date of publication and mention of the grant of the patent: **22.04.2026**
(21) Application number: 19855929.6
(22) Date of filing: 27.08.2019
(51) Int. Cl.: A61M 5/50, A61M 5/00, A61M 5/142, A61M 5/168, A61M 5/172, A61M 5/14

(54) **UTILIZING PRESSURE MEASUREMENTS TO DETECT REUSE OF PATIENT LINES**
VERWENDUNG VON DRUCKMESSUNGEN ZUR ERKENNUNG DER WIEDERVERWENDUNG VON PATIENTENLEITUNGEN
UTILISATION DE MESURES DE PRESSION POUR DÉTECTER LA RÉUTILISATION DE CONDUITES DE PATIENT

(30) Priority: 28.08.2018 US 201862723718 P
(43) Date of publication of application: 07.07.2021
(73) Proprietor: Bayer HealthCare, LLC, Whippany, NJ 07981 (US)
(72) Inventor: PARKER, Samantha, Pittsburgh, Pennsylvania 15239 (US)
(74) Representative: BIP Patents
(86) International application number: PCT/US2019/048337
(87) International publication number: WO 2020/046929

(56) References cited:
- US-A1- 2008 183 131
- US-A1- 2008 183 131
- US-A1- 2010 262 078
- US-A1- 2010 262 078
- US-A1- 2014 299 542
- US-A1- 2017 035 965
- US-A1- 2017 343 446

## Description

### BACKGROUND OF THE DISCLOSURE

### Field of the Disclosure

The present disclosure relates generally to detecting reuse of single-use disposable fluid path set components in fluid injector systems. More specifically, the present disclosure relates to fluid injector systems, computer program products, and methods for utilizing pressure measurements to detect reuse of single-use disposable fluid path set components in such fluid injector systems.

### Description of the Related Art

In many medical diagnostic and therapeutic procedures, a medical practitioner, such as a physician, injects a patient with one or more medical fluids. In recent years, a number of medical fluid delivery systems for pressurized injection of fluids, such as a contrast solution (often referred to simply as "contrast"), a flushing agent or diluent, such as saline, and other medical fluids, have been developed for use in procedures such as angiography, computed tomography (CT), ultrasound, magnetic resonance imaging (MRI), positron emission tomography (PET), and other molecular imaging procedures. In general, these medical fluid delivery systems are designed to deliver a preset amount of fluid at a preset flow rate.

In some injection procedures, the medical practitioner places a catheter or needle into a vein or artery of the patient. The catheter or needle is connected to either a manual or an automatic fluid injector system by way of tubing and a connector that interfaces with the fluid injector system. Automatic fluid injector systems typically include at least one syringe connected to at least one fluid injector having, for example, a powered linear piston. The at least one syringe includes, for example, a source of contrast and/or a source of flushing fluid. The medical practitioner enters settings into an electronic control system of the fluid injector for a fixed volume of contrast and/or saline and a fixed rate of injection for each. A single-use disposable set connector and associated tubing are connected to the fluid injector system for delivering one or more fluids to the patient.
Systems and methods related to air bubble removal from tubing are disclosed for example in US 2008/0183131 A1 or US 2010/0262078 A1. US 2008/0183131 A1 discloses a system and method including a fluid communications network that sends priming and waste fluid to a waste bag, obviating the presence of open fluid containers in an operating room or catheter lab. The fluid communications network is constructed and arranged to allow nearly automated priming and bubble removal, thereby reducing the possibility of operator caused errors in set-up and reducing the time required for set-up. The fluid communications network is useable for attachment to a balloon catheter for inflation thereof. In order to provide greater control and automation of the inflation of the balloon catheter, a conversion kit is provided that can be used to convert an existing automatic injector into an injector useable for automatically controlling the small amount of injection fluid typically associated with balloon catheters. US 2010/0262078 A1 discloses a system and method for adjusting insulin infusion volume based on air in an infusion set tube including inputting at least one location and length of an air bubble along the infusion set tube. An air bubble volume is determined in the infusion set tube. An insulin infusion volume is determined based on a desired insulin infusion. A determination is then made whether the desired insulin infusion would include the air bubble volume based on the inputted location and length of the air bubble. The air bubble volume is added to the insulin infusion volume to make an adjusted insulin infusion volume if the insulin infusion volume includes the air bubble.

To prevent contamination between patients and medical devices, each single-use disposable set connector and associated tubing is ideally replaced between patients. However, users may be inclined to reuse single-use disposable set connectors to save time and cost, an unhygienic and potentially dangerous practice.

While various manual and automatic fluid delivery systems are known in the medical field, improved multi-fluid delivery systems adapted for use in medical diagnostic and therapeutic procedures where one or more fluids are supplied to a patient during such procedures continue to be in demand. In particular, there exists a need for fluid delivery systems and single-use disposable set connectors which encourage and enforce safe and hygienic work practices.

### SUMMARY OF DISCLOSURE

To accomplish these objectives, the present invention provides a fluid injector system according to claim 1 and a computer program product according to claim 14. Further embodiments are defined in the dependent claims.

In accordance with one example, the exemplary administration line may be one of (i) an unused administration line and, as a result thereof, the extant fluid therein is the gas and the predetermined pressure profile thereby represents the pressure expected to be generated by the priming fluid during the priming of the unused administration line as the gas therein is completely displaced thereby over the course of the priming operation; and (ii) a previously used administration line and, as a result thereof, the extant fluid therein is at least partially a liquid and the predetermined pressure profile thereby represents the pressure expected to be generated by the priming fluid during the priming of the previously used administration line as the liquid therein is completely displaced thereby over the course of the priming operation. When the exemplary administration line is the unused administration line, upon the comparison resulting in a correlation within a specified tolerance between the distinct pressure profile and the predetermined pressure profile, the at least one processor is configured to determine that the subject administration line contained the gas as the extant fluid.

In accordance with other examples, the operation may further include: upon the comparison resulting in the correlation within the specified tolerance between the distinct pressure profile and the predetermined pressure profile, generating an alert indicating that the subject administration line had not been used prior to being primed with the at least one fluid during the priming operation. The operation may further include: upon the comparison resulting in the correlation within the specified tolerance between the distinct pressure profile and the predetermined pressure profile, permitting performance of the injection protocol.

In accordance with other examples, the subject administration line, like the exemplary administration line, may include at least one check valve precluding fluid flow in a proximal direction. The comparison resulting in the correlation within the specified tolerance between the distinct pressure profile and the predetermined pressure profile may include at least one of: identifying at least one pressure inflection point in the distinct pressure profile caused by the at least one fluid having passed through the at least one check valve in the subject administration line that correlates to at least one pressure inflection point corresponding thereto in the predetermined pressure profile caused by the priming fluid having passed through the at least one check valve in the exemplary administration line; normalizing the distinct pressure profile about a steady state value thereof, normalizing the predetermined pressure profile about a steady state value thereof, and determining that an area under a curve of the normalized distinct pressure profile correlates to an area under a curve of the predetermined pressure profile; and normalizing the distinct pressure profile about a steady state value thereof, normalizing the predetermined pressure profile about a steady state value thereof, and determining that each point along the distinct pressure profile correlates within a specified tolerance to corresponding points on the predetermined pressure profile.

In accordance with other examples, the exemplary administration line may be the previously used administration line, and upon the comparison resulting in a correlation within a specified tolerance between the distinct pressure profile and the predetermined pressure profile, the at least one processor may be configured to determine that the subject administration line contained the liquid as the extant fluid. The operation may further include: upon the comparison resulting in the correlation within the specified tolerance between the distinct pressure profile and the predetermined pressure profile, generating an alert indicating that the subject administration line had been used prior to being primed with the at least one fluid during the priming operation.

In accordance with other examples, the subject administration line, like the exemplary administration line, may include at least one check valve precluding fluid flow in a proximal direction. The comparison resulting in the correlation within the specified tolerance between the distinct pressure profile and the predetermined pressure profile may include at least one of: identifying at least one pressure inflection point in the distinct pressure profile caused by the at least one fluid having passed through the at least one check valve in the subject administration line that correlates to at least one pressure inflection point corresponding thereto in the predetermined pressure profile caused by the priming fluid having passed through the at least one check valve in the exemplary administration line; normalizing the distinct pressure profile about a steady state value thereof, normalizing the predetermined pressure profile about a steady state value thereof, and determining that an area under a curve of the normalized distinct pressure profile correlates to an area under a curve of the predetermined pressure profile; and normalizing the distinct pressure profile about a steady state value thereof, normalizing the predetermined pressure profile about a steady state value thereof, and determining that each point along the distinct pressure profile correlates within a specified tolerance to corresponding points on the predetermined pressure profile.

In accordance with other examples, the at least one fluid used in the priming of the subject administration line may include at least one of (i) a diluent, (ii) a contrast medium and (iii) a mixture of the contrast medium and the diluent. The determining the distinct pressure profile may include measuring a motor current of the at least one drive component.

In accordance with other examples, the subject administration line, like the exemplary administration line, may include a first check valve precluding fluid flow in a proximal direction and a second check valve precluding flow in the proximal direction. The second check valve may be located distally of the first check valve. During the priming of the subject administration line, the operation further may include: identifying a first pressure inflection point in the distinct pressure profile caused by the at least one fluid having passed through the first check valve in the subject administration line; and identifying at least one of: a second pressure inflection point in the distinct pressure profile caused by the at least one fluid having passed through the second check valve in the subject administration line; and a steady state portion of the distinct pressure profile over which the pressure generated within the subject administration line remains substantially constant. While comparing the distinct pressure profile to the predetermined pressure profile, the operation may further include: determining, based on a result of the comparison, that the administration line is fully primed.

In accordance with other examples, the subject administration line, like the exemplary administration line, may include a single check valve located in a distal end of the subject administration line. The single check valve may preclude fluid flow in a proximal direction. During the priming of the subject administration line, the operation further may include identifying at least one of: a pressure inflection point in the distinct pressure profile caused by the at least one fluid having passed through the single check valve in the subject administration line; and a steady state portion of the distinct pressure profile over which the pressure generated within the subject administration line remains substantially constant. While comparing the distinct pressure profile to the predetermined pressure profile, the operation may further include: determining, based on a result of the comparison, a presence of a fluid path set component connected to a distal end of the administration line.

Subject-matter of the present invention is also a computer program product for detecting multiple uses of an administration line according to claim 14.

In accordance with one example, the exemplary administration line may be one of (i) an unused administration line and, as a result thereof, the extant fluid therein is the gas and the predetermined pressure profile thereby represents the pressure expected to be generated by the priming fluid during the priming of the unused administration line as the gas therein is completely displaced thereby over the course of the priming operation; and (ii) a previously used administration line and, as a result thereof, the extant fluid therein is at least partially a liquid and the predetermined pressure profile thereby represents the pressure expected to be generated by the priming fluid during the priming of the previously used administration line as the liquid therein is completely displaced thereby over the course of the priming operation. When the exemplary administration line is the unused administration line, upon the comparison resulting in a correlation within a specified tolerance between the distinct pressure profile and the predetermined pressure profile, the one or more instructions, when executed by the at least one processor, may cause the at least one processor to determine that the subject administration line contained the gas as the extant fluid.

In accordance with other examples, the one or more instructions, when executed by the at least one processor, may cause the at least one processor to perform a further operation including: upon the comparison resulting in the correlation within the specified tolerance between the distinct pressure profile and the predetermined pressure profile, generating an alert indicating that the subject administration line had not been used prior to being primed with the at least one fluid during the priming operation. The one or more instructions, when executed by the at least one processor, may cause the at least one processor to perform a further operation including: upon the comparison resulting in the correlation within the specified tolerance between the distinct pressure profile and the predetermined pressure profile, permitting performance of the injection protocol.

In accordance with other examples, the subject administration line, like the exemplary administration line, may include at least one check valve precluding fluid flow in a proximal direction. The comparison resulting in the correlation within the specified tolerance between the distinct pressure profile and the predetermined pressure profile may include at least one of: identifying, with the at least one processor, at least one pressure inflection point in the distinct pressure profile caused by the at least one fluid having passed through the at least one check valve in the subject administration line that correlates to at least one pressure inflection point corresponding thereto in the predetermined pressure profile caused by the priming fluid having passed through the at least one check valve in the exemplary administration line; normalizing, with the at least one processor, the distinct pressure profile about a steady state value thereof; normalizing, with the at least one processor, the predetermined pressure profile about a steady state value thereof; and determining, with the at least one processor, that an area under a curve of the normalized distinct pressure profile correlates to an area under a curve of the predetermined pressure profile; and normalizing the distinct pressure profile about a steady state value thereof, normalizing the predetermined pressure profile about a steady state value thereof, and determining that each point along the distinct pressure profile correlates within a specified tolerance to corresponding points on the predetermined pressure profile.

In accordance with other examples, the exemplary administration line may be the previously used administration line, and upon the comparison resulting in a correlation within a specified tolerance between the distinct pressure profile and the predetermined pressure profile, the one or more instructions, when executed by the at least one processor, may cause the at least one processor to determine that the subject administration line contained the liquid as the extant fluid. The one or more instructions, when executed by the at least one processor, may cause the at least one processor to perform a further operation including: upon the comparison resulting in the correlation within the specified tolerance between the distinct pressure profile and the predetermined pressure profile, generating an alert indicating that the subject administration line had been used prior to being primed with the at least one fluid during the priming operation.

In accordance with other examples, the subject administration line, like the exemplary administration line, may include at least one check valve precluding fluid flow in a proximal direction. The comparison resulting in the correlation within the specified tolerance between the distinct pressure profile and the predetermined pressure profile may include at least one of: identifying, with the at least one processor, at least one pressure inflection point in the distinct pressure profile caused by the at least one fluid having passed through the at least one check valve in the subject administration line that correlates to at least one pressure inflection point corresponding thereto in the predetermined pressure profile caused by the priming fluid having passed through the at least one check valve in the exemplary administration line; normalizing, with the at least one processor, the distinct pressure profile about a steady state value thereof; normalizing, with the at least one processor, the predetermined pressure profile about a steady state value thereof; and determining, with the at least one processor, that an area under a curve of the normalized distinct pressure profile correlates to an area under a curve of the predetermined pressure profile; and normalizing, with the at least one processor, the distinct pressure profile about a steady state value thereof, normalizing, with the at least one processor, the predetermined pressure profile about a steady state value thereof, and determining, with the at least one processor, that each point along the distinct pressure profile correlates within a specified tolerance to corresponding points on the predetermined pressure profile.

In accordance with other examples, the at least one fluid used in the priming of the subject administration line may include at least one of (i) a diluent, (ii) a contrast medium and (iii) a mixture of the contrast medium and the diluent. The determining the distinct pressure profile may include measuring, with the at least one processor, a motor current of the at least one drive component.

In accordance with other examples, the subject administration line, like the exemplary administration line, may include a first check valve precluding fluid flow in a proximal direction and a second check valve precluding flow in the proximal direction. The second check valve may be located distally of the first check valve. During priming of the subject administration line, the operation further may include: identifying a first pressure inflection point in the distinct pressure profile caused by the at least one fluid having passed through the first check valve in the subject administration line; and identifying at least one of: a second pressure inflection point in the distinct pressure profile caused by the at least one fluid having passed through the second check valve in the subject administration line; and a steady state portion of the distinct pressure profile over which the pressure generated within the subject administration line remains substantially constant. While comparing the distinct pressure profile to the predetermined pressure profile, the operation further may include: determining, based on a result of the comparison, that the administration line is fully primed.

In accordance with other examples, the subject administration line, like the exemplary administration line, may include a single check valve located in a distal end of the subject administration line. The single check valve may preclude fluid flow in a proximal direction. During the priming of the subject administration line, the operation further may include identifying at least one of: a pressure inflection point in the distinct pressure profile caused by the at least one fluid having passed through the single check valve in the subject administration line; and a steady state portion of the distinct pressure profile over which the pressure generated within the subject administration line remains substantially constant. While comparing the distinct pressure profile to the predetermined pressure profile, the operation further may include: determining, based on a result of the comparison, a presence of a fluid path set component connected to a distal end of the administration line.

These and other features and characteristics of fluid injector systems, computer program products, and methods of operation and functions of the related elements of structures and the combination of parts and economies of manufacture, will become more apparent upon consideration of the following description and the appended claims with reference to the accompanying drawings, all of which form a part of this specification. It is to be expressly understood, however, that the drawings are for the purpose of illustration and description only.

### BRIEF DESCRIPTION OF THE DRAWINGS

**FIG. 1** is a perspective view of a multi-fluid delivery system, according to one example;
**FIG. 2** is a schematic view of various fluid paths within the multi-fluid delivery system of **FIG. 1****;**
**FIG. 3A** is a perspective view of a connection interface prior to connecting a single-use disposable set connector with a multi-fluid delivery system;
**FIG. 3B** is a perspective view of the connection interface of **FIG. 3A** showing the single-use disposable set connector connected with the multi-fluid delivery system;
**FIG. 4A** is a perspective view of a single-use disposable set connector in accordance with one example;
**FIG. 4B** is a cross-sectional view of the single-use disposable set connector shown in **FIG. 4A****;**
**FIG. 4C** is a cross-sectional view of the single-use disposable set connector shown in **FIG. 4A** connected to a port of a multi-fluid delivery system;
**FIG. 5** is a perspective view of the single-use disposable set connector shown in **FIG. 4C** with a portion of the multi-fluid delivery system and the multi-patient disposable set cutaway;
**FIG. 6** is a schematic view of an electronic control system of a multi-fluid injection system in accordance with another example;
**FIG. 7** is a graphical representation of a predetermined pressure profile and a distinct pressure profile representative of the single-use disposable set connector of **FIGS. 4A-5****;**
**FIG. 8** is a step sequence diagram of a method of determining the nature of an extant fluid in the single-use disposable set connector of **FIGS. 4A-5****;**
**FIG. 9** is a graphical representation of the predetermined pressure profile and the distinct pressure profile of **FIG. 7** normalized about steady state portions thereof;
**FIG. 10** is a step sequence diagram of a method of determining that the single-use disposable set connector of **FIGS. 4A-5** is fully primed;
**FIG. 11** is a graphical representation of the predetermined pressure profile of **FIG. 7** and a modified distinct pressure profile representative of the single-use disposable set connector of **FIGS. 4A-5** having an additional fluid path set component attached thereto;
**FIG. 12** is a graphical representation of the predetermined pressure profile of **FIG. 7** and various predetermined pressure profiles representative of the single-use disposable set connector of **FIGS. 4A-5** at various ages and use states; and
**FIG. 13** is a graphical representation of the predetermined pressure profile of **FIG. 7** and various predetermined pressure profiles representative of the single-use disposable set connector of **FIGS. 4A-5** coupled with varying extant fluids in a multi-patient disposable set of the multi-fluid delivery system of **FIG. 1****.**

### DETAILED DESCRIPTION

For purposes of the description hereinafter, the terms "upper", "lower", "right", "left", "vertical", "horizontal", "top", "bottom", "lateral", "longitudinal", and derivatives thereof shall relate to the disclosure as it is oriented in the drawing figures. When used in relation to a syringe, a single-use disposable set connector, and/or a fluid path set component, the term "proximal" refers to a portion of the syringe, the single-use disposable set connector, and/or the fluid path set component nearest to an injector when the syringe, the single-use disposable set connector, and/or the fluid path set component is oriented for connecting to the injector. The term "distal" refers to a portion of the syringe, the single-use disposable set connector, and/or the fluid path set component farthest away from the injector when the syringe, the single-use disposable set connector, and/or the fluid path set component is oriented for connecting to the injector.

As used herein, the term "correlation" and derivatives thereof refers to an observed and/or calculated association(s) between data. Correlation may include, for example, a relative difference between two or more data points, a statistical relationship between two or more data sets, and combinations thereof. As used herein, the term "specified tolerance" refers to a predetermined percentage difference, a predetermined standard deviation, a predetermined statistical correlation coefficient, and/or the like. For example, a first value may exhibit correlation within a specified tolerance of a second value if the first value is within a predetermined percentage difference (e.g., within 10%) of the second value. Similarly, a data point may exhibit correlation within a specified tolerance of a data set if the data point falls within a predetermined standard deviation (e.g., within one standard deviation) of the data set. Similarly, a first curve may exhibit correlation within a specified tolerance of a second curve if the first curve includes specific features (e.g. inflection points) within a predetermined range (e.g., within 10 sampling time intervals) of like features of the second curve. Similarly, a first curve may exhibit correlation within a specified tolerance of a second curve if an area under the first curve is within a predetermined percentage difference (e.g., within 10%) of an area under the second curve.

As used herein, the term "normalize" and derivatives thereof refers to adjusting individual values of a data set to a common scale. For example, normalizing may refer to dividing all values of a data set by a value corresponding to a steady state condition, such that each value of the normalized data set is referenced to the steady state condition.

As used herein, the term "and/or" refers to both or either of two stated possibilities. For example, when used with reference to "first and/or second predetermined pressure profile", this phrase refers to a combination of both of the first and the second predetermined pressure profile, or one of the first predetermined pressure profile and the second predetermined pressure profile.

All numbers used in the specification and claims are to be understood as being modified in all instances by the term "about". The terms "about", "approximately", and "substantially" means a range of plus or minus ten percent of the stated value.

As used herein, the term "at least one of" is synonymous with "one or more of". For example, the phrase "at least one of A, B, and C" means any one of A, B, and C, or any combination of any two or more of A, B, and C. For example, "at least one of A, B, and C" includes one or more of A alone; or one or more B alone; or one or more of C alone; or one or more of A and one or more of B; or one or more of A and one or more of C; or one or more of B and one or more of C; or one or more of all of A, B, and C. Similarly, as used herein, the term "at least two of" is synonymous with "two or more of". For example, the phrase "at least two of D, E, and F" means any combination of any two or more of D, E, and F. For example, "at least two of D, E, and F" includes one or more of D and one or more of E; or one or more of D and one or more of F; or one or more of E and one or more of F; or one or more of all of D, E, and F.

It is also to be understood that the specific devices and processes illustrated in the attached drawings, and described in the following specification, are simply exemplary examples of the disclosure. Hence, specific dimensions and other physical characteristics related to the examples disclosed herein are not to be considered as limiting.

Although the present disclosure is described primarily in the context of the MEDRAD^{®} Centargo CT Injection System, it will be apparent to persons of ordinary skill in the art that the present disclosure can be applied to a variety of injection systems inclusive of their associated disposables (e.g., syringes, tubing, etc.). Examples of such injection systems include the MEDRAD^{®} Stellant CT Injection System, the MEDRAD^{®} Stellant FLEX CT Injection System, the MEDRAD^{®} MRXperion MR Injection System and the MEDRAD^{®} Mark 7 Arterion Injection System offered by Bayer HealthCare LLC.

Referring now to the drawings in which like reference characters refer to like parts throughout the several views thereof, the present disclosure in some aspects and examples thereof is generally directed to a multi-fluid medical injector/injection system **100** (hereinafter "fluid injector system **100")** having a multi-patient disposable set (MUDS) **130** configured for delivering fluid to a patient using a single-use disposable set (SUDS) **190** connector. The fluid injector system **100** includes multiple components as individually described herein. Generally, the fluid injector system **100** has a powered injector or other administration device and a fluid delivery set intended to be associated with the injector to deliver one or more fluids from one or more multi-dose containers under pressure into a patient, as described herein. The various devices, components, and features of the fluid injector system **100** and the fluid delivery set associated therewith are likewise described in detail herein.

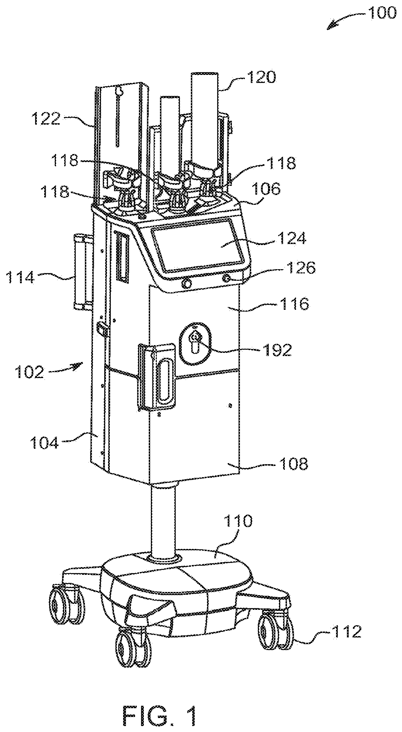

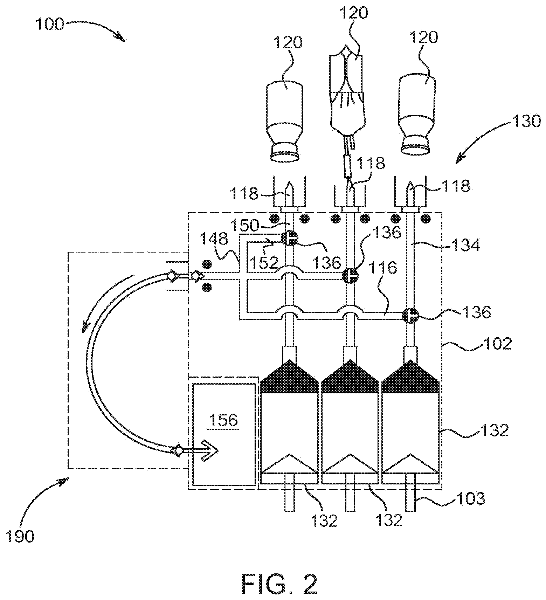

With reference to **FIG. 1****,** the fluid injector system **100** includes an injector housing **102** having opposed lateral sides **104,** a distal or upper end **106,** and a proximal or lower end **108.** In some examples, the housing **102** may be supported on a base **110** having one or more wheels **112** for rotatable and movable support of the housing **102** on a floor surface. The one or more wheels **112** may be lockable to prevent the housing **102** from inadvertently moving once positioned at a desired location. At least one handle **114** may be provided to facilitate moving and positioning the fluid injector system **100.** In other examples, the housing **102** may be removably or non-removably secured to a fixed surface, such as a floor, ceiling, wall, or other structure. The housing **102** encloses the various mechanical drive components, electrical and power components necessary to drive the mechanical drive components, and control components, such as electronic memory and electronic control devices (hereinafter electronic control device(s)), used to control operation of reciprocally movable piston elements **103** (shown in **FIG. 2****)** associated with the fluid injector system **100** described herein. Such piston elements **103** may be reciprocally operable via electro-mechanical drive components such as a ball screw shaft driven by a motor, a voice coil actuator, a rack-and-pinion gear drive, a linear motor, and the like. In some examples, at least some of the mechanical drive components, electrical and power components, and control components may be provided on the base **110.**

With continued reference to **FIG. 1****,** the fluid injector system **100** has at least one door **116** that encloses at least some of the MUDS, the mechanical drive components, electrical and power components, and control components. The door **116** is desirably movable between an open position and a closed position (shown in **FIG. 1****).** In some examples, the door **116** may be lockable.

The fluid injector system **100** further includes at least one bulk fluid connector **118** for connection with at least one bulk fluid source **120.** In some examples, a plurality of bulk fluid connectors **118** may be provided. For example, as shown in **FIG. 1****,** three bulk fluid connectors **118** may be provided in a side-by-side or other arrangement. In some examples, the at least one bulk fluid connector **118** may be a spike configured for removably connecting to the at least one bulk fluid source **120,** such as a vial, a bottle, or a bag. The at least one bulk fluid connector **118** may have a reusable or non-reusable interface with each new bulk fluid source **120.** The at least one bulk fluid connector **118** may be formed on the multi-patient disposable set, as described herein. The at least one bulk fluid source **120** may be configured for receiving a medical fluid, such as saline, contrast solution, or other medical fluid, for delivery to the fluid injector system **100.** The housing **102** may have at least one support member **122** for supporting the at least one bulk fluid source **120** once it is connected to the fluid injector system **100.**

With continued reference to **FIG. 1****,** the fluid injector system **100** includes one or more user interfaces **124,** such as a graphical user interface (GUI) display window. The user interface **124** may display information pertinent to a fluid injection procedure involving the fluid injector system **100,** such as current flow rate, fluid pressure, and volume remaining in the at least one bulk fluid source **120** connected to the fluid injector system **100** and may be a touch screen GUI that allows an operator to input commands and/or data for operation of the fluid injector system **100.** While the user interface **124** is shown on the injector housing **102,** such user interface **124** may also be in the form of a remote display that is wired or wirelessly linked to the housing **102** and control and mechanical elements of fluid injector system **100.** In some examples, the user interface **124** may be a tablet computer that is detachably connected to the housing **102** and is in wired or wirelessly linked communication with the housing **102** and control and mechanical elements of the fluid injector system **100.** Additionally, the fluid injector system **100** and/or user interface **124** may include at least one control button **126** for tactile operation by an attendant operator of the fluid injector system **100.** In certain examples, the at least one control button **126** may be part of a keyboard for inputting commands and/or data by the operator. The at least one control button **126** may be hard-wired to the electronic control device(s) associated with the fluid injector system **100** to provide direct input to the electronic control device(s). The at least one control button **126** may also be a graphical part of the user interface **124,** such as a touch screen. In either arrangement, the at least one control button **126** desirably provides certain individual control features to the attendant operator of the fluid injector system **100,** such as, but not limited to: (1) acknowledging that a multi-patient disposable set has been loaded or unloaded; (2) locking/unlocking the multi-patient disposable set; (3) filling/purging the fluid injector system **100;** (4) inputting information and/or data related to the patient and/or injection procedure; (5) preloading the fluid injector system **100;** and (6) initiating/stopping an injection procedure. The user interface **124** and/or any electronic processing units associated with the fluid injector system **100** may be wired or wirelessly connected to an operation and/or data storage system such as a hospital network system.

With reference to **FIG. 2****,** the fluid injector system **100** includes a MUDS **130** that is removably connected to the fluid injector system **100** for delivering one or more fluids from the one or more bulk fluid sources **120** to the patient. Examples and features of the MUDS are further described in International Patent Application Publication No. WO 2016/112163, filed on January 7, 2016 and entitled "Multiple Fluid Delivery System with Multi-Use Disposable Set and Features Thereof". The MUDS **130** may include one or more syringes or pumps **132.** In some examples, the number of syringes **132** may correspond to the number of bulk fluid sources **120.** For example, with reference to **FIG. 2****,** the MUDS **130** has three syringes **132** in a side-by-side arrangement such that each syringe **132** is fluidly connectable to one or more of the bulk fluid sources **120.** In some examples, one or two bulk fluid sources **120** may be connected to one or more syringes **132** of the MUDS **130.** Each syringe **132** may be fluidly connectable to one of the bulk fluid sources **120** by a corresponding bulk fluid connector **118** and an associated MUDS fluid path **134.** The MUDS fluid path **134** may have a spike element that connects to the bulk fluid connector **118.** In some examples, the bulk fluid connector **118** may be provided directly on the MUDS **130.**

With further reference to **FIG. 2****,** the MUDS **130** is removably connectable to the housing **102** of the fluid injector system **100.** As will be appreciated by one having ordinary skill in the art, it may be desirable to construct at least a portion of the MUDS **130** from a clear medical grade plastic in order to facilitate visual verification that a fluid connection has been established with the fluid injector system **100.** Visual verification is also desirable for confirming that no air bubbles are present within various fluid connections. Alternatively, at least a portion of the MUDS **130** and/or door **116** may include windows (not shown) for visualization of the connection between various components. Various optical sensors (not shown) may also be provided to detect and verify the connections. Additionally, various lighting elements (not shown), such as light emitting diodes (LEDs), may be provided to actuate one or more optical sensors and indicate that a suitable connection has been established between the various components.

With specific reference to **FIG. 2****,** a schematic view of various fluid paths of the fluid injector system **100** is provided. The MUDS **130** may include one or more valves **136,** such as stopcock valves, for controlling which medical fluid or combinations of medical fluids are withdrawn from the multi-dose bulk fluid source **120** and/or are delivered to a patient through each syringe **132.** In some examples, the one or more valves **136** may be provided on a distal end of the plurality of syringes **132** or on a manifold **148.** The manifold **148** may be in fluid communication via valves **136** and/or syringes **132** with a first end of the MUDS fluid path **134** that connects each syringe **132** to the corresponding bulk fluid source **120.** The opposing second end of the MUDS fluid path **134** may be connected to the respective bulk fluid connector **118** that is configured for fluidly connecting with the bulk fluid source **120.** Depending on the position of the one or more valves **136,** fluid may be drawn into the one or more syringes **132** or it may be delivered from the one or more syringes **132.** In a first position, such as during the filling of the syringes **132,** the one or more valves **136** are oriented such that fluid flows from the bulk fluid source **120** into the desired syringe **132** through a fluid inlet line **150,** such as a MUDS fluid path. During the filling procedure, the one or more valves **136** are positioned such that fluid flow through one or more fluid outlet lines **152** or the manifold **148** is blocked. In a second position, such as during a fluid delivery procedure, fluid from one or more syringes **132** is delivered to the manifold **148** through the one or more fluid outlet lines **152** or syringe valve outlet ports. During the delivery procedure, the one or more valves **136** are positioned such that fluid flow through one or more fluid inlet lines **150** is blocked. The one or more valves **136,** fluid inlet lines **150,** and/or fluid outlet lines **152** may be integrated into the manifold **148.** The one or more valves **136** may be selectively positioned to the first or second position by manual or automatic handling. For example, the operator may position the one or more valves **136** into the desired position for filling or fluid delivery. In other examples, at least a portion of the fluid injector system **100** is operable for automatically positioning the one or more valves **136** into a desired position for filling or fluid delivery based on input by the operator, as described herein.

With continued reference to **FIG. 2****,** in some examples, the fluid outlet line **152** may also be connected to a waste reservoir **156** of the fluid injector system **100.** The waste reservoir **156** is desirably separate from the syringes **132** to prevent contamination. In some examples, the waste reservoir **156** is configured to receive waste fluid expelled from the syringes **132** during, for example, a flushing, priming, or preloading operation. The waste reservoir **156** may be removable from the housing **102** in order to dispose of the contents of the waste reservoir **156.** In other examples, the waste reservoir **156** may have a draining port (not shown) for emptying the contents of the waste reservoir **156** without removing the waste reservoir **156** from the housing **102**. In some examples, the waste reservoir **156** is provided as a separate component from the MUDS **130.**

Having generally described the components of the fluid injector system **100** and the MUDS **130,** the structure and method of use of a single-use disposable set (SUDS) **190** and its interaction with MUDS **130** will now be described. Hereinafter, the SUDS **190** may be referred to as the administration line.

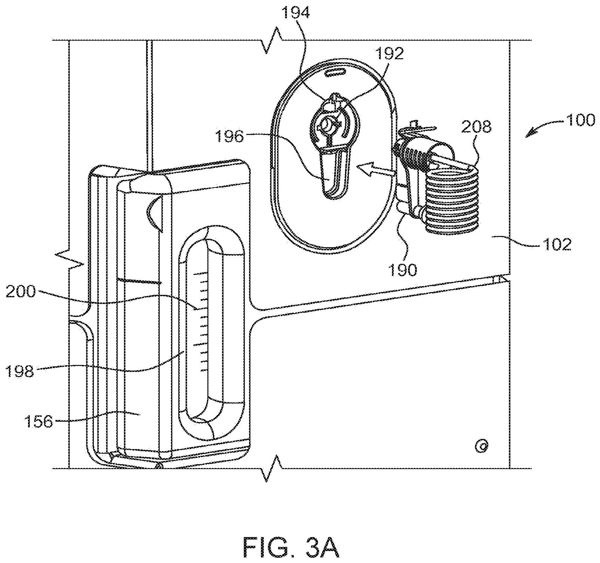

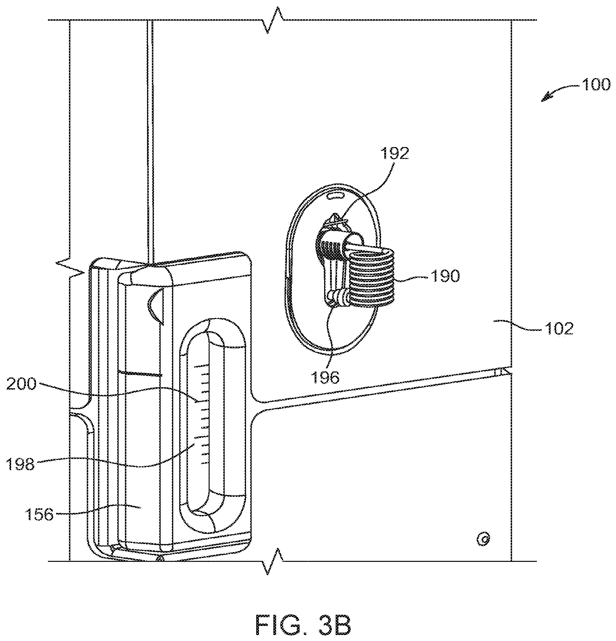

With reference to **FIGS. 3A** **and** **3B****,** the fluid injector system **100** has a connection port **192** that is configured to form a releasable fluid connection with at least a portion of the SUDS **190.** In some examples, the connection port **192** may be formed on the MUDS **130.** The connection port **192** may be shielded by at least a portion of the housing **102** of the fluid injector system **100.** For example, recessing the connection port **192** within the interior of the housing **102** may preserve the sterility of the connection port **192** by preventing or limiting a user or patient from touching and contaminating the portions of the connection port **192** that contact the fluid to be injected into the patient. In some examples, the connection port **192** is recessed within an opening **194** formed on the housing **102** of the fluid injector system **100,** or the connection port **192** may have a shielding structure (not shown) that surrounds at least a portion of the connection port **192.** In other examples, the connection port **192** may be formed directly on the housing **102** and connected to the MUDS **130** by a fluid path (not shown). As described herein, the SUDS **190** may be connected to the connection port **192,** formed on at least a portion of the MUDS **130** and/or the housing **102.** Desirably, the connection between the SUDS **190** and the connection port **192** is a releasable connection to allow the SUDS **190** to be selectively disconnected from the connection port **192 (****FIG. 3A****)** and connected to the connection port **192 (****FIG. 3B****).** In some examples, the SUDS **190** may be disconnected from the connection port **192** and disposed after each fluid delivery procedure, and a new SUDS **190** may be connected to the connection port **192** for a subsequent fluid delivery procedure.

With continued reference to **FIGS. 3A** **and** **3B****,** a waste inlet port **196** may be provided separately from the connection port **192.** The waste inlet port **196** is in fluid communication with the waste reservoir **156.** In some examples, the waste reservoir **156** is provided separately from the SUDS **190** such that the fluid from the waste inlet port **196** can be delivered to the waste reservoir **156.** At least a portion of the SUDS **190** may be releasably connected to or associated with the waste inlet port **196** for introducing waste fluid into the waste reservoir **156** during, for example, a priming operation that expels air from the SUDS **190.** The waste reservoir **156** may have a viewing window **198** with indicia **200,** such as graduated markings, that indicate the fill level of the waste reservoir **156.**

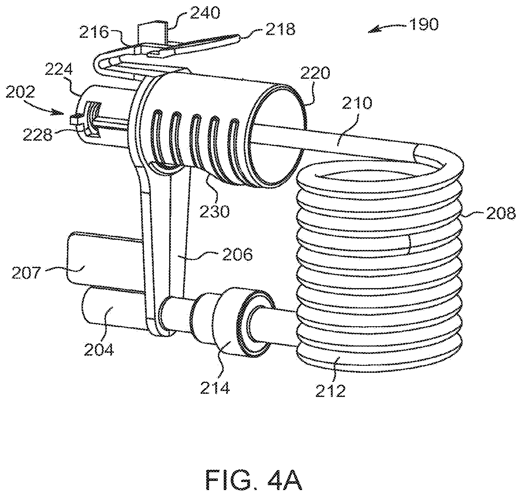

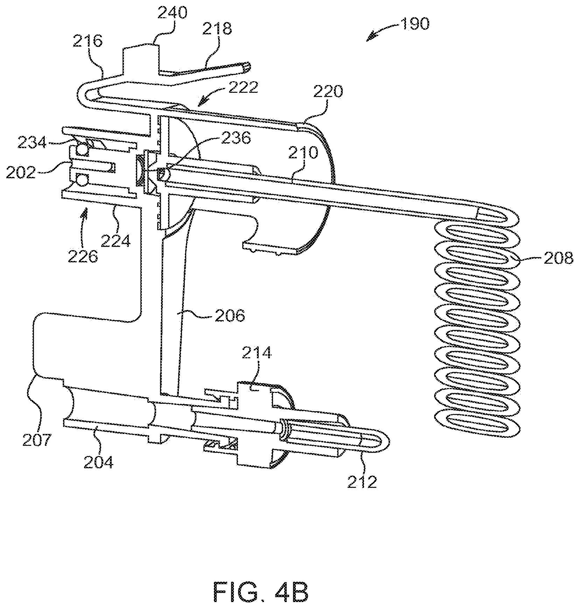

With reference to **FIG. 4A****,** the SUDS **190** has a fluid inlet port **202** that is configured for releasable connection with the connection port **192** (shown in **FIG. 3A****).** The fluid inlet port **202** receives fluid delivered from the fluid injector system **100.** The fluid inlet port **202** is desirably a hollow, tubular structure, as shown in **FIG. 4B****.** The SUDS **190** further has a waste outlet port **204** that is configured for releasable connection or association with the waste inlet port **196** (shown in **FIG. 3A****).** The waste outlet port **204** receives waste fluid and delivers such waste fluid to the waste reservoir **156** during, for example, a priming or flushing operation of the SUDS **190.** The waste outlet port **204** is desirably a hollow, tubular structure, as shown in **FIG. 4B****.** The waste outlet port **204** may be connected to, inserted into, or located in the waste inlet port **196** so that the waste fluid may flow through the waste inlet port **202** and continue into the waste reservoir **156.** The fluid inlet port **202** and the waste outlet port **204** may be spaced apart from each other by a spacer **206.** In some examples, the spacer **206** is dimensioned to position the fluid inlet port **202** and the waste outlet port **204** for alignment with the connection port **192** and the waste inlet port **196,** respectively. It is noted that the SUDS **190** is shown in **FIG. 4A** in a state after removal from packaging (not shown). Prior to use, the SUDS **190** is desirably packaged in a pre-sterilized, sealed package that protects the SUDS **190** from contamination with airborne or surface-borne contaminants. Alternatively, the sealed package and the SUDS **190** may be sterilized after packaging.

The SUDS **190** desirably has an asymmetrical structure, so that the user can only attach the SUDS **190** to the MUDS **130** in one orientation. In this manner, the user is prevented from attaching the fluid inlet port **202** to the waste inlet port **196.** In some examples, a fin **207** may be provided on at least a portion of the SUDS **190** to prevent erroneous insertion of the SUDS **190** in the connection port **192.** In certain examples, the fin **207** may be formed on the spacer **206** proximate to the waste outlet port **204.** In this manner, the fin **207** may interfere with the incorrect insertion of the SUDS **190** into the connection port **192.** Structures and shapes other than the fin **207** may be used to prevent erroneous insertion of the SUDS **190** into the connection port **192.**

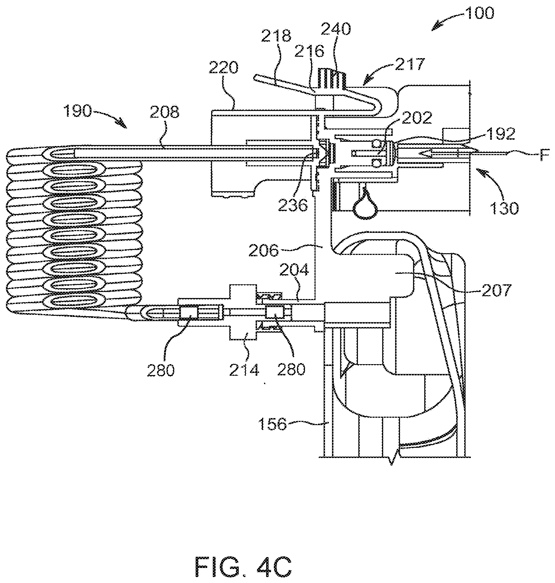

In some examples, tubing **208,** may be connected at its proximal end **210** to the fluid inlet port **202.** The tubing **208** is configured to deliver fluid received from the fluid inlet port **202.** The distal end **212** of the tubing **208** may have a connector **214,** which may include a one-way check valve, that is configured for connection with the waste outlet port **204** or a fluid path connected to the patient (not shown). The tubing **208** may be made from a flexible material, such as a medical grade plastic material, that allows the tubing **208** to be coiled. The connector **214** may be a luer-lock connector (either a male luer-lock connector or a female luer-lock connector depending on the desired application) or other medical connector configuration. In some examples, the connector **214** may include a one-way check valve **280** therein, as shown in **FIGS. 4B** and **4C****,** to prevent backflow of fluid into the tubing **208** from a catheter or other component attached to the connector **214.**

With continued reference to **FIG. 4A****,** the SUDS **190** may have a locking tab **216** that is configured for selectively locking the SUDS **190** with the fluid injector system **100** depending on the engagement of the locking tab **216** with at least a portion of the fluid injector system **100.** In some examples, the locking tab **216** may be a flexible tab that is deflectable between an engaged position and a disengaged position by deflecting at least a portion of the locking tab **216.** The locking tab **216** may have a pressing surface **218** that, when pressed, causes the locking tab **216** to be deflected from the engaged position to the disengaged position for insertion and removal of the SUDS **190** from the fluid injector system **100.** In some examples, the locking tab **216** may be configured for releasable locking engagement with a receiving slot **217** on the MUDS **130** (shown in **FIG. 4C****).**

With reference to **FIG. 4B****,** the SUDS **190** may have a first annular skirt **224** extending circumferentially around a proximal end **226** of the fluid inlet port **202** and a second annular skirt **220** extending circumferentially around a distal end **222** of the fluid inlet port **202.** The first and second annular skirts **224, 220** surround the fluid inlet port **202** to prevent inadvertent contact and contamination. The first annular skirt **224** may have one or more recesses **228** (shown in **FIG. 4A****)** extending through a sidewall thereof. The one or more recesses **228** may provide a locking interface with a corresponding locking element (not shown) on the fluid injector system **100.** The second annular skirt **220** may have at least one indentation **230** (shown in **FIG. 4A****)** to facilitate grasping and handling of the SUDS **190.** In some examples, the second annular skirt **220** may have a textured surface having one or more ribs to facilitate gripping and handling of the SUDS **190.**

With continued reference to **FIG. 4B****,** at least one annular seal **234** may be provided around the proximal end **226** of the fluid inlet port **202.** The at least one annular seal **234** may seal the fluid inlet port **202** to prevent fluid from leaking through the SUDS **190.** The at least one annular seal **234** may provide a fluid seal between the SUDS **190** and the MUDS **130** when they are fluidly connected with one another to allow fluid to flow from the MUDS **130** to the SUDS **190** without leaking. A one-way check valve **236** may be provided within a lumen of the fluid inlet port **202** to prevent fluid from flowing in a reverse direction from the SUDS **190** into the MUDS **130.**

With reference to **FIG. 4C****,** the SUDS **190** shown in **FIG. 4A** is shown connected to the fluid injector system **100.** While **FIG. 4C** illustrates the connection port **192** formed on the MUDS **130,** in other examples, the connection port **192** may be formed on a portion of the housing **102** (shown in **FIG. 1****).** The fluid inlet port **202** of the SUDS **190** is connected to the connection port **192** to establish a fluid path in a direction of arrow **F** shown in **FIG. 4C****.** Fluid passing through the fluid inlet port **202** flows through the one-way valve **236** and into the tubing **208.** Any fluid that may drip from the interface between the fluid inlet port **202** and the connection port **192** is collected in the waste reservoir **156.** The waste reservoir **156** may be shaped to collect any fluid that may drip from the SUDS **190** when it is removed from the MUDS **130**. Additionally, when the SUDS **190** is connected to the connection port **192,** the outlet of the waste outlet port **204** is positioned within the waste inlet port **196** such that waste fluid from the tubing **208** may be discharged into the waste reservoir **156.** The spacer **206** may define an insertion stop surface to define the depth of insertion of the SUDS **190** into the connection port **192.**

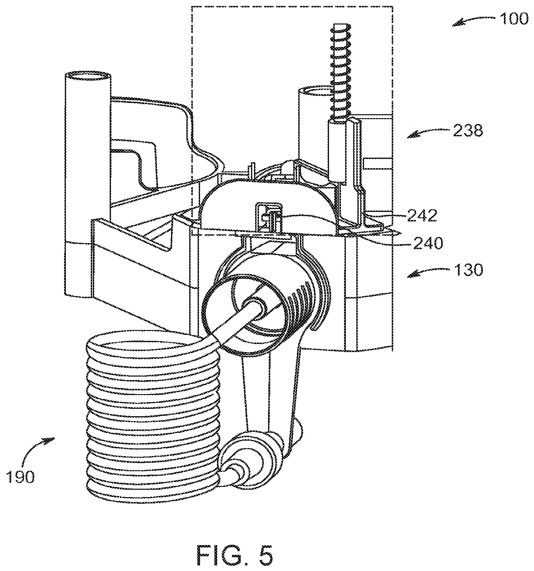

With reference to **FIG. 5****,** the fluid injector system **100** may have a sensor system **238** adapted to identify when the SUDS **190** is in fluid communication with the MUDS **130.** The sensor system **238** may include at least one sensing element, such as a sensor fin **240,** on the SUDS **190** and a corresponding sensor **242** on the fluid injector system **100** or MUDS **130.** The sensor **242** may be configured to detect the presence and absence of the at least one sensor fin **240** or other sensing element. In some examples, the sensing element, such as the at least one sensor fin **240,** is formed on the locking tab **216** of the SUDS **190,** such as shown in **FIG. 4A****.** In other examples, the sensing element, such as the at least one sensor fin **240,** may be formed on any portion of the SUDS **190.** The sensor **242** may be an optical sensor that is seated and secured within a respective mount formed on the housing **102** of the fluid injector system **100.** As will be appreciated by those versed in the field of powered medical fluid injectors, the sensor **242** may be electronically coupled to an electronic control device used to discretely control operation of the fluid injector system, such as the operation of the one or more piston elements, based, at least in part, on input from the sensor **242.** The sensing element, such as the sensor fin **240,** may have one or more reflective surfaces that reflect visible or infrared light to be detected by the sensor **242.** In other examples, mechanical interaction between the sensing element and the sensor **242** may be used.

In some examples, the SUDS **190** may further include reuse prevention features. For example, the SUDS **190** may include one or more breakable sensor elements, tabs, or structures that fold or break when the SUDS **190** is removed from the MUDS **130.** Absence of these features may prevent reinsertion and reuse of the SUDS **190** after removal. In this manner, it can be assured that the SUDS **190** is only used for one fluid delivery procedure.

Other examples and features of the SUDS **190** are described in U.S. Patent Application Publication No. 2016/0331951, filed July 7, 2016 and entitled *"Single-Use Disposable Set Connector".*

Having generally described the components of the fluid injector system **100,** the MUDS **130,** and the SUDS **190,** a method of operation of using the SUDS **190** will now be described in detail. In use, a medical technician or user removes the disposable SUDS **190** from its packaging (not shown) and inserts the fluid inlet port **202** into the connection port **192** on the MUDS **130.** As described above, the SUDS **190** must be inserted in the correct orientation such that the fluid inlet port **202** is aligned for connection with the connection port **192** and the waste outlet port **204** is aligned for connection with the waste inlet port **196.** The SUDS **190** may be secured to the MUDS **130** by inserting the locking tab **216** into the receiving slot **217** on the MUDS **130.** Once the SUDS **190** is securely connected to the MUDS **130,** for example as sensed by the sensor **242,** the fluid injector system **100** (shown in **FIG. 1****)** draws fluid into one or more of the plurality of syringes **132** of the MUDS **130** and performs an automatic priming or flushing operation for removing air from the MUDS **130** and the SUDS **190.** During such priming or flushing operation, fluid from the MUDS **130** is injected through the connection port **192** and into the tubing **208** of the SUDS **190.** The fluid flows through the tubing **208,** the connector **214** and through the waste outlet port **204** and into the waste reservoir **156.** Once the automatic priming or flushing operation is completed, the tubing **208** may optionally be preloaded with an injection protocol by injecting fluid(s) from the MUDS **130** through the connection port **192.** Additional details of the preloading operation will be described later in greater detail. After the automatic priming or flushing operation and, optionally, the preloading operation are completed, the medical technician disconnects the connector **214** from the waste outlet port **204.** The connector **214** may then be connected to the patient via a catheter, vascular access device, needle, or additional fluid path set to facilitate fluid delivery to the patient. Once the fluid delivery is completed, the SUDS **190** is disconnected from the patient and the MUDS **130** by disengaging the locking tab **216** of the SUDS **190** from the receiving slot **217** on the MUDS **130.** The medical technician may then dispose of the SUDS **190.** In certain examples, removing the SUDS **190** from the MUDS **130** causes reuse prevention features (not shown) to activate, thereby preventing reinsertion and reuse of the SUDS **190.**

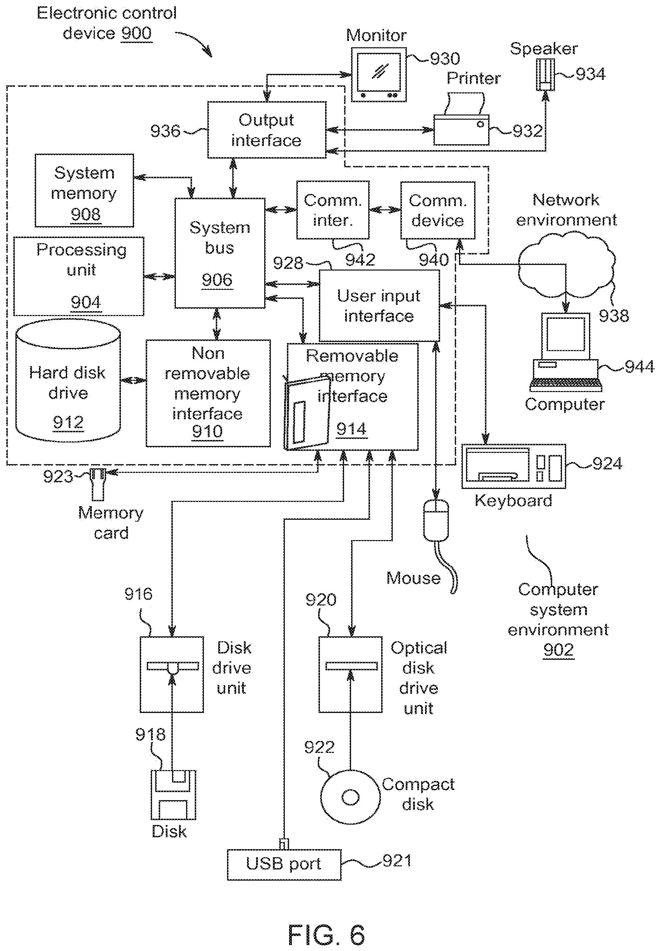

With reference to **FIG. 6****,** an electronic control device **900** may be associated with the fluid injector system **100** to control the filling and delivery operations. In some examples, the electronic control device **900** may control the operation of various valves, piston members, and other elements to effect a desired filling or delivery procedure. For example, the electronic control device **900** may include a variety of discrete computer-readable media components. For example, this computer-readable media may include any media that can be accessed by the electronic control device **900,** such as volatile media, non-volatile media, removable media, non-removable media, transitory media, non-transitory media, etc. As a further example, this computer-readable media may include computer storage media, such as media implemented in any method or technology for storage of information, such as computer-readable instructions, data structures, program modules, or other data; random access memory (RAM), read-only memory (ROM), electrically erasable programmable read-only memory (EEPROM), flash memory, or other memory technology; CD-ROM, digital versatile disks (DVDs), or other optical disk storage; magnetic cassettes, magnetic tape, magnetic disk storage, or other magnetic storage devices; or any other medium which can be used to store the desired information and which can be accessed by the electronic control device **900.** Further, this computer-readable media may include communications media, such as computer-readable instructions, data structures, program modules, or other data in a modulated data signal, such as a carrier wave or other transport mechanism and include any information delivery media, wired media (such as a wired network and a direct-wired connection), and wireless media (such as acoustic signals, radio frequency signals, optical signals, infrared signals, biometric signals, bar code signals, etc.). Of course, combinations of any of the above should also be included within the scope of computer-readable media.

The electronic control device **900** further includes a system memory **908** with computer storage media in the form of volatile and non-volatile memory, such as ROM and RAM. A basic input/output system (BIOS) with appropriate computer-based routines assists in transferring information between components within the electronic control device **900** and is normally stored in ROM. The RAM portion of the system memory **908** typically contains data and program modules that are immediately accessible to or presently being operated on by a processor **904,** e.g., an operating system, application programming interfaces, application programs, program modules, program data, and other instruction-based computer-readable codes.

With continued reference to **FIG. 6****,** the electronic control device **900** may also include other removable or non-removable, volatile or non-volatile, transitory or non-transitory computer storage media products. For example, the electronic control device **900** may include a non-removable memory interface **910** that communicates with and controls a hard disk drive **912,** e.g., a non-removable, non-volatile magnetic medium; and a removable, non-volatile memory interface **914** that communicates with and controls a magnetic disk drive unit **916** (which reads from and writes to a removable, non-volatile magnetic disk **918),** an optical disk drive unit **920** (which reads from and writes to a removable, non-volatile optical disk **922,** such as a CD ROM), a Universal Serial Bus (USB) port **921** for use in connection with a removable memory card, etc. However, it is envisioned that other removable or non-removable, volatile or non-volatile computer storage media can be used in an exemplary computing system environment **902,** including, but not limited to, magnetic tape cassettes, DVDs, digital video tape, solid state RAM, solid state ROM, etc. These various removable or non-removable, volatile or non-volatile magnetic media are in communication with the processor **904** and other components of the electronic control device **900** via a system bus **906.** The drives and their associated computer storage media, discussed above and illustrated in **FIG. 6****,** provide storage of operating systems, computer-readable instructions, application programs, data structures, program modules, program data, and other instruction-based, computer-readable code for the electronic control device **900** (whether duplicative or not of this information and data in the system memory **908).**

A user may enter commands, information, and data into the electronic control device **900** through certain attachable or operable input devices, such as the user interface **124** shown in **FIG. 1****,** via a user input interface **928.** A variety of such input devices may be utilized, e.g., a microphone, a trackball, a joystick, a touchpad, a touch-screen, a scanner, etc., including any arrangement that facilitates the input of data and information to the electronic control device **900** from an outside source. As discussed, these and other input devices are often connected to the processor **904** through the user input interface **928** coupled to the system bus **906,** but may be connected by other interface and bus structures, such as a parallel port, game port, or a USB. Still further, data and information can be presented or provided to a user in an intelligible form or format through certain output devices, such as a monitor **930** (to visually display this information and data in electronic form), a printer **932** (to physically display this information and data in print form), a speaker **934** (to audibly present this information and data in audible form), etc. All of these devices are in communication with the electronic control device **900** through an output interface **936** coupled to the system bus **906.** It is envisioned that any such peripheral output devices be used to provide information and data to the user.

The electronic control device **900** may operate in a network environment **938** through the use of a communications device **940,** which is integral to the electronic control device **900** or remote therefrom. This communications device **940** is operable by and in communication with the other components of the electronic control device **900** through a communications interface **942.** Using such an arrangement, the electronic control device **900** may connect with or otherwise communicate with one or more remote computers, such as a remote computer **944,** which may be a personal computer, a server, a router, a network personal computer, a peer device, or other common network nodes, and typically includes many or all of the components described above in connection with the electronic control device **900.** Using appropriate communication devices **940,** e.g., a modem, a network interface or adapter, etc., the computer **944** may operate within and communicate through a local area network (LAN) and a wide area network (WAN), but may also include other networks such as a virtual private network (VPN), an office network, an enterprise network, an intranet, the Internet, etc.

As used herein, the electronic control device **900** includes or is operable to execute appropriate custom-designed or conventional software to perform and implement the processing steps of the method and system of the present disclosure, thereby forming a specialized and particular computing system. Accordingly, the method and system may include one or more electronic control devices **900** or similar computing devices having a computer-readable storage medium capable of storing computer-readable program code or instructions that cause the processor **904** to execute, configure, or otherwise implement the methods, processes, and transformational data manipulations discussed hereinafter in connection with the present disclosure. Still further, the electronic control device **900** may be in the form of a personal computer, a personal digital assistant, a portable computer, a laptop, a palmtop, a mobile device, a mobile telephone, a server, or any other type of computing device having the necessary processing hardware to appropriately process data to effectively implement the fluid injector system, the computer program product and the computer-implemented method of the present disclosure.

It will be apparent to one skilled in the relevant arts that the system may utilize databases physically located on one or more computers which may or may not be the same as their respective servers. For example, programming software on the electronic control device **900** can control a database physically stored on a separate processor of the network or otherwise.

In some examples, the electronic control device **900** may be programmed so that automatic refill occurs based upon a preprogrammed trigger minimum volume in the respective syringes **132.** For example, when the volume of fluid remaining in at least one of the syringes **132** is less than a programmed volume, a syringe refill procedure is automatically initiated by the electronic control device **900.** The electronic control device 900 associated with the fluid injector system **100** may determine that the preprogrammed trigger minimum volume has been reached by tracking the fluid volume dispensed from the respective syringes **132** during operation of the fluid injector system **100.** Alternatively, fluid level sensors may be incorporated into the fluid injector system **100** and inputs from these fluid level sensors may be provided to the electronic control device **900** so that the electronic control device **900** may determine when the preprogrammed trigger minimum volume has been reached in at least one of the syringes **132.** The fill volume and rate of refill can be preprogrammed in the electronic control device **900.** The automatic refill procedure can be stopped either automatically by the electronic control device **900** or may be manually interrupted. In addition, an automatic refill procedure may be initiated when, at the completion of a fluid injection procedure, there is not enough fluid in at least one of the syringes **132** to perform the next programmed fluid injection procedure.

During a refill procedure it is possible that one or more of the bulk fluid sources **120** associated with the respective syringes **132** may become empty (e.g., initially lack sufficient fluid to complete a full refill of the one or more syringes **132).** A replacement bulk fluid source **120** is, therefore, necessary and replacement of such bulk fluid source **120** is desirably made quickly. The fluid injector system **100** may have an indicator, such as an audible and/or visual indicator, to indicate to the operator that a change of the bulk fluid source **120** is necessary before the fluid injector system **100** may be used.

As described above, the fluid injector system **100** may automatically or manually prime the MUDS **130** and the SUDS **190** once the SUDS **190** is securely connected to the MUDS **130,** for example, as sensed by the sensor **242**. During such a priming operation, saline, or another suitable diluent, is injected from the MUDS **130** through the connection port **192,** into the tubing **208** of the SUDS **190,** and into the waste reservoir **156.** Flow of the priming fluid toward the waste reservoir **156** purges extant fluid from the fluid injector system **100** by forcing any extant fluid in SUDS **190** and/or in the manifold **148** of the MUDS **130** out the distal end **212** of the tubing **208.** The priming fluid thus replaces any extant fluid in the SUDS **190** with the priming fluid. During the priming operation, various components of the fluid injector system **100** may communicate with the electronic control device **900** to continuously or intermittently monitor a pressure generated during delivery of the priming fluid from the MUDS **130** through the SUDS **190.** By monitoring this pressure, the electronic control device **900** can determine various characteristics of the SUDS **190** and associated components. In various aspects or examples of the present disclosure, the electronic control device **900** may be utilized to determine whether the SUDS **190** has been previously used, whether the SUDS **190** has been fully primed, the presence of an additional fluid path set component connected to the connector **214** of the SUDS **190,** the length of the SUDS **190,** and/or the age of the SUDS **190.** These and other aspects and examples of the present disclosure will be discussed in detail herein.

In some aspects or examples, the electronic control device **900** may be utilized to determine whether the SUDS **190** has been previously used based on an extant fluid displaced from the SUDS **190** during the priming thereof. If unused, the SUDS **190** may be initially filled with a gas, such as air or another gas injected into the SUDS **190** during manufacture and/or packaging, whereas a used SUDS **190** may be filled with a liquid, such as a residual medical liquid from a previously performed injection protocol. The electronic control device **900** may determine whether the extant fluid displaced from the SUDS **190** during priming was a gas, indicating that the SUDS **190** is unused, or a liquid, indicating that the SUDS **190** was previously used. The determination of whether the extant, displaced fluid is a gas or liquid may be based on a pressure profile generated during priming of the SUDS **190.** The pressure profile may be obtained by measuring the pressure generated as a result of displacing extant fluid from the SUDS **190** at predetermined time intervals as the priming fluid is injected through the SUDS **190** during the priming operation of the SUDS **190.** Hereinafter, this pressure profile, which represents actual measured pressure of the priming operation over time, will be referred to as the "distinct pressure profile".

To determine whether the extant fluid displaced from the SUDS **190** during priming was a gas or a liquid, the electronic control device **900** may compare the distinct pressure profile to a predetermined pressure profile. The predetermined pressure profile is representative of pressure expected to be generated within an exemplary SUDS by the priming fluid over a course of a priming operation performed on the exemplary SUDS. In particular, the predetermined pressure profile may be representative of the pressure expected to be generated in an unused SUDS during an identical priming operation to that performed on the actual subject SUDS **190.** The predetermined pressure profile may be obtained through pressure measurement of an exemplary SUDS known to be unused.

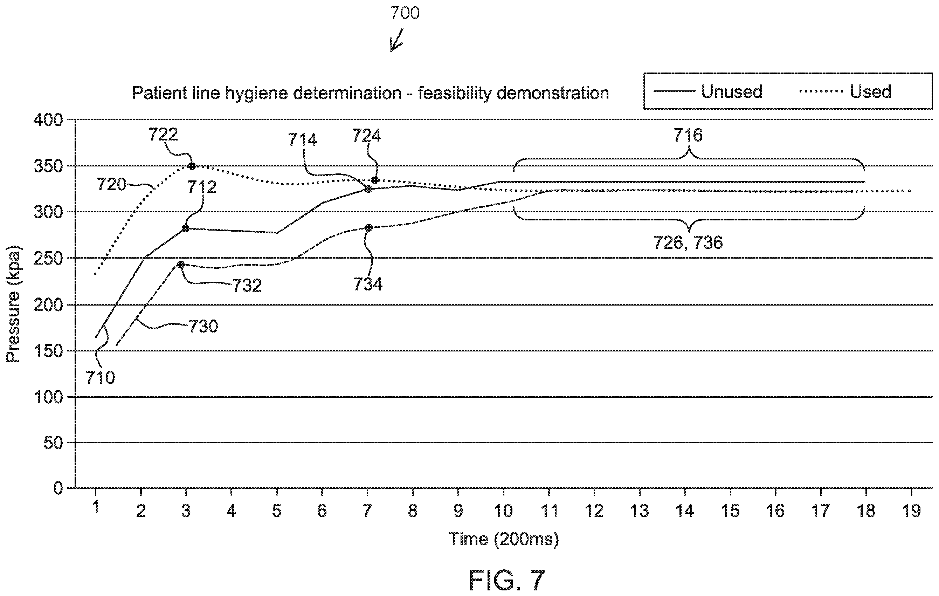

Correlation between the distinct pressure profile and the predetermined pressure profile is indicative of whether the SUDS **190,** prior to the priming operation, contained gas as the extant fluid or liquid as the extant fluid. Predetermined pressure profiles for various exemplary SUDS may be presented graphically to facilitate interpretation and comparison of a predetermined pressure profile and a distinct pressure profile. **FIG. 7** illustrates a graph **700,** including a graphical representation of a first predetermined pressure profile **710** and a graphical representation of a second predetermined pressure profile **720,** with time on the x-axis and pressure on the y-axis. In the example shown in **FIG. 7****,** time is presented in units of 200 milliseconds (ms) and pressure is presented in units of kilopascals (kPa). For the first predetermined pressure profile **710,** the graphed pressure corresponds to expected pressure for an exemplary SUDS known to be unused, whereas for the second predetermined pressure profile **720,** the graphed pressure corresponds to expected pressure for an exemplary SUDS known to have been used. **FIG. 7** further includes a graphical representation of a distinct pressure profile **730** generated during priming of the SUDS **190.** The graphed time begins at 0 ms, corresponding to initiation of the priming operation, and extends through completion of the priming operation.

Various events over the course of the priming operation may be appreciated from the graph **700** of **FIG. 7** by identifying specific pressure values and/or changes in pressure values over time. A first inflection point **712** of the first predetermined pressure profile **710** may correspond to a time at which the priming fluid passes through the one-way check valve **236** of the fluid inlet port **202** of the exemplary SUDS. In particular, the pressure change at a first inflection point **712** indicates the one-way check valve **236** being opened in response to an accumulation of fluid pressure in the MUDS **130** leading into the exemplary SUDS. Similarly, a second inflection point **714** of the predetermined pressure profile **710** may correspond to a time at which the priming fluid passes through the one-way check valve **280** in the connector **214** at the distal end of the exemplary SUDS. The pressure change at the second inflection point **714** indicates the one-way check valve **280** being opened in response to an accumulation of fluid pressure in the exemplary SUDS. Subsequent to the second inflection point **714,** pressure fluctuation of the predetermined pressure profile **710** settles to a steady state portion **716** corresponding to a time interval over which the priming fluid flows freely through both of the one way check valves **236, 280** of the exemplary SUDS.

With continued reference to **FIG. 7****,** the graphical representation of the second predetermined pressure profile **720** may include a first inflection point **722,** a second inflection point **724,** and a steady state portion **726.** The first inflection point **722** may correspond to a time at which the priming fluid passes through the one-way check valve **236** of the fluid inlet port **202** of the exemplary SUDS, the second inflection point **724** may correspond to a time at which the priming fluid passes through the one-way check valve **280** in the connector **214** of the exemplary SUDS, and the steady state portion **726** may correspond to a time interval over which the priming fluid flows freely through both of the one way check valves **236, 280** of the exemplary SUDS.

With continued reference to **FIG. 7****,** the graphical representation of the distinct pressure profile **730** may include a first inflection point **732,** a second inflection point **734,** and a steady state portion **736.** The first inflection point **732** may correspond to a time at which the priming fluid passes through the one-way check valve **236** of the fluid inlet port **202** of the SUDS **190,** the second inflection point **734** may correspond to a time at which the priming fluid passes through the one-way check valve **280** in the connector **214** of the SUDS **190,** and the steady state portion **736** may correspond to a time interval over which the priming fluid flows freely through both of the one way check valves **236, 280** of the SUDS **190.**

In **FIG. 7****,** the exemplary SUDS represented by the first predetermined pressure profile **710** is unused, meaning that the extant fluid contained in the exemplary SUDS prior to priming was a gas. In contrast, the exemplary SUDS represented by the second predetermined pressure profile **720** was previously used, meaning that the extant fluid contained in the exemplary SUDS prior to priming was at least partly a liquid. The distinct pressure profile **730** is illustrative of an unused SUDS **190.**

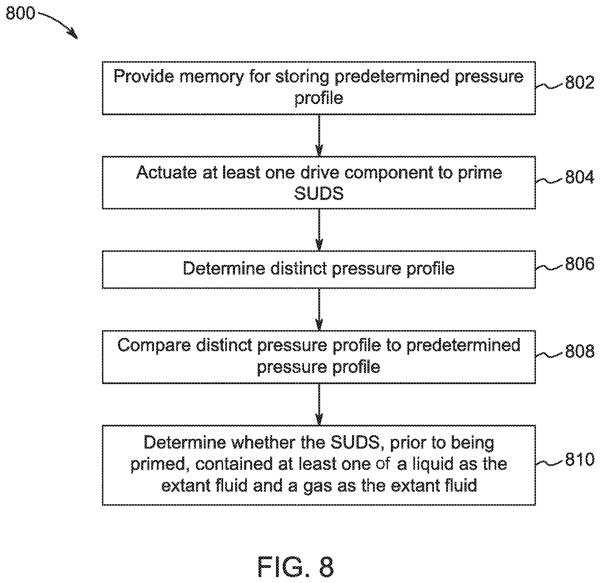

Having generally described the characteristics of the pressure profiles in general, a method **800** of detecting reuse of the SUDS **190** in accordance with some aspects and examples of the present disclosure will be described with reference to **FIG. 8****.** At step **802,** the method **800** may include providing a memory for storing the predetermined pressure profile **710.** The memory may be, for example, the hard disk drive **912** or another memory device integral with or in communication with the electronic control device **900.** In particular, the memory may store, as a database, the individual pressure measurements and corresponding time indices of the first and second predetermined pressure profiles **710, 720.**

With continued reference to **FIG. 8****,** at step **804,** the method **800** may further include actuating at least one drive component, such as one or more of the piston elements **103,** of the fluid injector system **100** to prime the SUDS **190.** The priming operation of step **804** may be performed substantially as described above, including the injection of saline, or another suitable diluent, from the MUDS **130** through the connection port **192,** into the tubing **208** of the SUDS **190,** and into the waste reservoir **156.**

With continued reference to **FIG. 8****,** at step **806,** the method **800** may further include determining the distinct pressure profile **730,** as described above, by measuring the pressure generated during the priming of the SUDS **190** during the priming operation of step **804.** In some aspects or examples, the pressures generated during the priming of the SUDS **190** represented in the distinct pressure profile **730** may be obtained and/or derived by measuring the current drawn by the electro-mechanical drive component, i.e. the motor current, of the piston element **103** injecting the priming fluid. In other aspects or examples, the pressures generated during the priming of the SUDS **190** represented in the distinct pressure profile **730** may be obtained and/or derived by one or more pressure transducers (not shown) mounted in the MUDS **130** and/or the SUDS **190** in fluid communication with the priming fluid. Other methods of pressure measurement may be appreciated by those of ordinary skill in the art. In some aspects or examples, each pressure measurement of the distinct pressure profile **730,** along with a corresponding time index indicating the relative time at which each pressure measurement occurred, may be stored in the hard disk drive **912** or another memory device integral with or in communication with the electronic control device **900.**

With continued reference to **FIG. 8****,** at step **808,** the method **800** may further include comparing the distinct pressure profile **730** to at least one of the first and second predetermined pressure profiles **710, 720.** Various methods of comparing the distinct pressure profile **730** to at least one of the first and second predetermined pressure profiles **710, 720** may be utilized. In some aspects or examples, the electronic control device **900** may compare the pressure measurements and/or time indices associated with the first inflection point **712,** the second inflection point **714,** and the steady state portion **716** of the first predetermined pressure profile **710** with the pressure measurements and/or time indices associated with the first inflection point **732,** the second inflection point **734,** and the steady state portion **736** of the distinct pressure profile **730.** Alternatively, or in addition, the electronic control device **900** may compare the pressure measurements and/or time indices associated with the first inflection point **722,** the second inflection point **724,** and the steady state portion **726** of the second predetermined pressure profile **720** with the pressure measurements and/or time indices associated with the first inflection point **732,** the second inflection point **734,** and the steady state portion **736** of the distinct pressure profile **730.** In some aspects or examples, the electronic control device **900** may compare specific pressure measurements at similar or identical time indices of the first and/or second predetermined pressure profiles **710, 720** and the distinct pressure profile **730.**

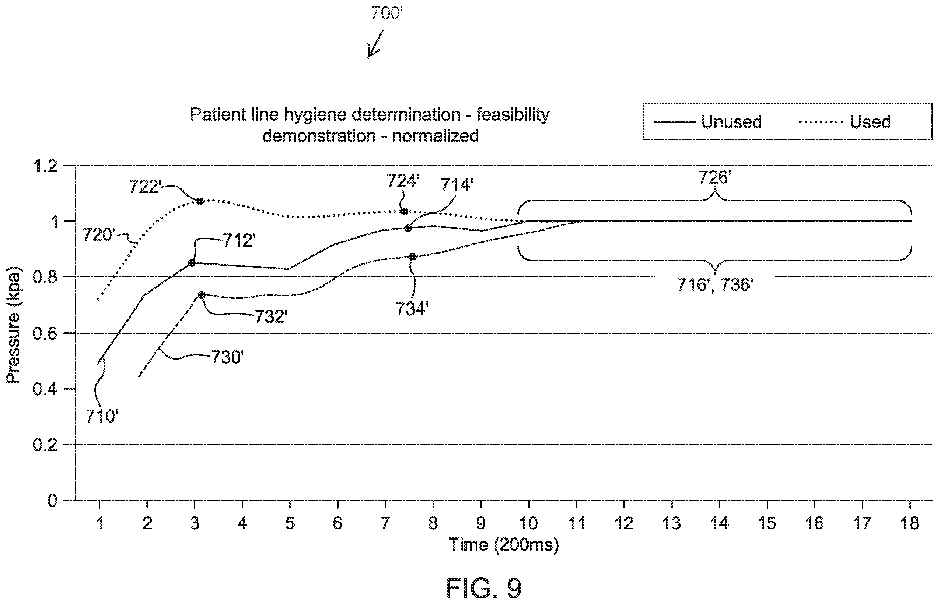

In some aspects or examples, step **808** may include normalizing, with the electronic control device **900,** one or both of the first and/or second predetermined pressure profiles **710, 720** and the distinct pressure profile **730** to facilitate comparison of the first and/or second predetermined pressure profile(s) **710, 720** and the distinct pressure profile **730.** In particular, the distinct pressure profile **730** may be normalized about the steady state portion **736** such that the pressure values within the steady state portion **736** are normalized to a value of one (1). The normalization may be performed by dividing each individual pressure measurement of the distinct pressure profile **730** by the steady state pressure value (e.g., an average of the values within the steady state portion **736).** The first and/or second predetermined pressure profile(s) **710, 720** may be normalized about the steady state portion **716** thereof in the same manner. **FIG. 9** shows a normalized graph **700'** of the graph of **FIG. 7****,** with normalized first and second predetermined pressure profiles **710', 720'** and a normalized distinct pressure profile **730'** being normalizations of the first and second predetermined pressure profiles **710, 720** and the distinct pressure profile **730** of **FIG. 7****.** Normalizing the first and/or second predetermined pressure profile(s) **710, 720** and the distinct pressure profile **730** facilitates comparison regardless of individual machine and/or the calibration differences of the fluid injector system **100** as the steady state portions **716', 726', 736'** are normalized about a value of one (1). With the first and/or second predetermined pressure profile(s) **710, 720** and the distinct pressure profile **730** normalized, the electronic control device **900** may, in some aspects or examples, compare an area under a curve of the normalized first and/or second predetermined pressure profile(s) **710', 720'** to an area under a curve of the normalized distinct pressure profile **730'.**

In some aspects or examples, step **808** may include comparing linear trendlines of the first and/or second predetermined pressure profile(s) **710, 720** and the distinct pressure profile **730** over a predefined duration. A portion of the predetermined pressure profile **730** between two predetermined time indices may be fitted with a best-fit straight line. A portion of the distinct pressure profile **730** between the same two time indices may similarly be fitted with a best-fit straight line. The best-fit straight lines of the first and/or second predetermined pressure profile(s) **710, 720** and the distinct pressure profile **730** may then be compared to determine characteristics of the SUDS **190** during the priming operation.

In other aspects or examples, step **808** may include a plurality of the comparison methods discussed above. Each of the comparison methods may be weighted as part of an overall comparison score from which conclusions regarding the first and/or second predetermined pressure profile(s) **710, 720** and the distinct pressure profile **730** may be drawn.

With continued reference to **FIG. 8****,** at step **810,** the method **800** may further include determining, based on at least one result of the comparison of step **808,** whether the SUDS **190,** prior to the priming thereof at step **804,** contained at least one of a liquid as the extant fluid and a gas as the extant fluid. Various methods may be used to make the determination at step **810.** In general, the electronic control device **900** may determine a correlation between one or more features and/or values of the first and/or second predetermined pressure profile(s) **710, 720** and the distinct pressure profile **730** compared at step **808.** If the correlation is within a specified tolerance, the electronic control device **900** may determine that the SUDS **190,** prior to being primed, contained an extant fluid of at least partly the same phase (e.g. liquid or gas) as the extant fluid contained in the exemplary SUDS.

In some aspects or examples, the electronic control device **900** may determine that the SUDS **190** contained a liquid or a gas as the extant fluid based on a correlation within a specified tolerance of the first inflection points **712, 722, 732** the second inflection points **714, 724, 734** and/or the steady state portions **716, 726, 736** of the first and/or second predetermined pressure profile **710, 720** and the distinct pressure profile **730.** In the example shown in **FIG. 7****,** the first inflection point **712** of the first predetermined pressure profile **710** occurs at approximately 275 kpa and 700 ms, whereas the first inflection point **722** of the second predetermined pressure profile **720** occurs at approximately 350 kpa and 750 ms. **FIG. 7** further shows that the first inflection point **732** of the distinct pressure profile **730** occurs at approximately 240 kpa and 650 ms. If the correlation (e.g., the difference between the pressures and/or the difference between the time indices) falls within a specified tolerance (e.g., 10% difference), the electronic control unit **900** may determine that the SUDS **190** contained extant fluid in the same phase (e.g. liquid or gas), prior to the priming operation, as the exemplary SUDS. In contrast, if the correlation falls outside the specified tolerance, the electronic control unit **900** may determine that the SUDS **190** contained extant fluid in a different phase, prior to the priming operation, than the exemplary SUDS. In this example, the correlation of the pressures and time indices of the first predetermined pressure profile **710** and the distinct pressure profile **730,** and/or the correlation of the pressures and time indices of the second predetermined pressure profile **720** and the distinct pressure profile **730** fall outside the specified tolerance, and therefore the electronic control unit **900** may determine that the SUDS **190** contained extant fluid in a different phase, prior to the priming operation, than the exemplary SUDS. As the exemplary SUDS contained air prior to the priming operation, the electronic control unit **900** may therefore determine that the SUDS **190** contained liquid prior to being primed and thus had been used in a previously performed injection protocol.

In some aspects or examples, step **810** may include determining that the SUDS 190 contained a liquid or a gas as the extant fluid based on a correlation of the normalized first and/or second predetermined pressure profile(s) **710', 720'** and the normalized distinct pressure profile **730'.** In particular, the electronic control device **900** may determine whether the correlation between the area under the curve of the normalized distinct pressure profile **730'** is within a specified tolerance to the area under the curve of the first and/or second predetermined pressure profile(s) **710', 720'.** If the electronic control device **900** makes a determination in the affirmative, the extant fluid contained in the SUDS **190** prior to priming was in the same phase as the extant fluid in the exemplary SUDS. If the electronic control device **900** makes a determination in the negative, the extant fluid contained in the SUDS **190** prior to priming was in a different phase than the extant fluid in the exemplary SUDS. Based on this determination, the electronic control unit **900** may determine whether the SUDS **190** has been used in a previously performed injection protocol.

In some aspects or examples, step **810** may include determining that the SUDS **190** contained a liquid or a gas as the extant fluid based on a correlation of best-fit lines of the first and second predetermined pressure profiles **710, 720** and the distinct pressure profile **730.** As with the above-described aspects, the electronic control unit **900** may determine that the SUDS **190** contained extant fluid in the same phase, prior to the priming operation, as the exemplary SUDS if the correlation falls within a specified tolerance. Conversely, the electronic control unit **900** may determine that the SUDS **190** contained extant fluid in a different phase, prior to the priming operation, than the exemplary SUDS if the correlation falls outside the specified tolerance. Based on this determination, the electronic control unit 900 may determine whether the SUDS **190** has been used in a previously performed injection protocol.

In other aspects or examples, step **810** may include a plurality of the above-described methods for determining whether the extant fluid contained in the SUDS **190,** prior to being primed, was a liquid or a gas. Each of these determinations may be weighted as part of an overall score from which conclusions regarding previous usage of the SUDS **190** may be drawn.

In some aspects or examples, the determination made at step **810** may be used to generate an alert indicating whether the SUDS **190** had been previously used. In particular, the electronic control unit **900** may generate the alert in response to determining that the SUDS **190,** prior to being primed, contained at least part liquid as the extant fluid and, therefore, was used in a previously performed injection protocol. The alert may be generated by the electronic control unit **900** in the form a visual, audio, tactile, or other sensory output configured to prompt the attention of a physician or other care provider. In some aspects or examples, the alert may be a graphic displayed on one or more user interfaces **124** of the fluid injector system **100,** a noise emitted from the speaker **934** of the fluid injector system **100,** or a combination thereof.

In some aspects or examples, the determination made at step **810** may be input into a compliance report generated by the electronic control unit **900.** The compliance report may be displayed on one or more user interfaces **124** of the fluid injector system **100** to provide visual feedback about compliance with hygienic practices, e.g., routine replacement of the SUDS **190.** The compliance report may also be stored in a compliance database for future analysis of hygienic practices. Further details of generating, displaying, and analyzing a compliance report utilizing the fluid injector system **100** are provided in International Patent Application No. PCT/US2019/026659, filed on April 9, 2019 and entitled *"System and Methods for Monitoring Hygiene Practices Associated with Use of Power Fluid Injector Systems".*

In some aspects or examples, the determination made at step **810** may prevent commencement of the injection protocol if the electronic control unit **900** determines that the SUDS **190** had been previously used. In particular, the electronic control unit **900** may prohibit commencement of an enabled inj ection protocol in response to determining that the SUDS **190,** prior to being primed, contained at least part liquid as the extant fluid and, therefore, was used in a previously performed injection protocol. Conversely, the electronic control unit **900** may permit commencement of the enabled injection protocol in response to determining that the SUDS **190,** prior to being primed, contained gas as the extant fluid and, therefore, is unused.

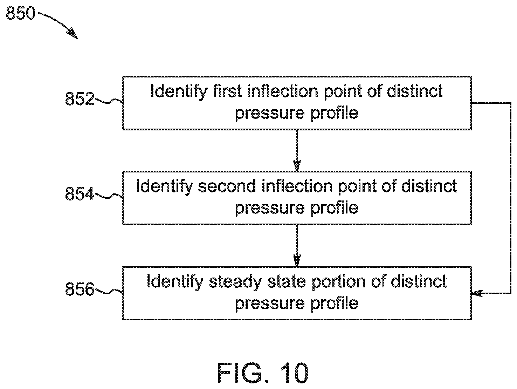

As noted above, monitoring the pressure generated during the priming operation may also be utilized to determine characteristics of the SUDS **190** other than the extant fluid contained therein. In some aspects or examples, the electronic control unit **900** may determine whether the SUDS **190** has been fully primed based on the distinct pressure profile **730.** **FIG. 10** shows a method **850** that may be used in determining whether the SUDS **190** has been fully primed. At step **852,** the method **850** may include identifying the first inflection point **732** in the distinct pressure profile **730** caused by the at least one fluid having passed through the one-way check valve **236** of the SUDS **190.** The electronic control unit **900** may identify the first inflection point **732** by identifying a sharp rise in pressure followed by a plateau in pressure due to opening of the check valve **236.** In some examples or aspects, the electronic control unit **900** may compare the distinct pressure profile **730** to the first and/or second predetermined pressure profile(s) **710, 720** and determine whether the first inflection point **732** of the distinct pressure profile **730** correlates within a specified tolerance to the first inflection point **712** of the first predetermined pressure profile **710** and/or the first inflection point **722** of the second predetermined pressure profile **720.**

With continued reference to **FIG. 10****,** the method **850** may further include, at step **854,** identifying the second inflection point **734** in the distinct pressure profile **730** caused by the at least one fluid having passed through the one-way check valve **280** of the SUDS **190.** The electronic control unit **900** may identify the second inflection point **734** by comparing the distinct pressure profile **730** to the first and/or second predetermined pressure profile(s) **710 ,720** and determining that the second inflection point **734** of the distinct pressure profile **730** correlates within a specified tolerance to the second inflection point **714, 724** of the first and/or second predetermined pressure profile(s) **710, 720.** If the electronic control unit **900** cannot identify the second inflection point **734** of the distinct pressure profile **730** correlated within the specified tolerance, the electronic control unit **900** may determine that the priming fluid has not reached the one-way check valve **280** and, thus, the SUDS **190** has not been fully primed.

With continued reference to **FIG. 10****,** the method **850** may further include, at step **856,** identifying the steady state portion **736** in the distinct pressure profile **730** caused by the priming fluid flowing freely after having passed through both one-way check valves **236, 280** of the SUDS **190.** The electronic control unit **900** may identify the steady state portion **736** by determining that the pressure is constant after a predetermined period of time during the priming operation. The electronic control unit **900** may identify the steady state portion **736** by comparing the distinct pressure profile **730** to the first and/or second predetermined pressure profile(s) **710, 720** and determining that the steady state portion **736** of the distinct pressure profile **730** correlates within a specified tolerance to the steady state portion **716, 726** of the first and/or second predetermined pressure profile(s) **710, 720.** If the electronic control unit **900** cannot identify the steady state portion **736** of the distinct pressure profile **730** as being so correlated within the specified tolerance, the electronic control unit **900** may determine that the priming fluid has not reached steady state and, thus, the SUDS **190** has not been fully primed. If the electronic control unit **900** identifies either the second inflection point **734** at step **854** or the steady state portion **736** at step **856,** the electronic control unit **900** may determine that the SUDS has been fully primed.

In some aspects or examples of the method **850,** only one of steps **854** and **856** may be performed. It is also noted that the method **850** as described above presumes that the SUDS **190** includes both one-way check valves **236** and **280.** However, in some aspects or examples, the one-way check valve **236** may be omitted, and the method **850** may be performed without step **852** since the first inflection point **732** would not be exhibited in the absence of the one-way check valve **236.**

In some aspects or examples, the electronic control unit **900** may determine a length of the SUDS **190** based on the distinct pressure profile **730.** The electronic control unit **900** may determine the elapsed time between the first inflection point **732** and the second inflection point **734** of the distinct pressure profile **730,** indicative of the time between the openings of the one-way check valves **236, 280.** The electronic control unit **900** may further determine the volume of fluid injected into the SUDS **190,** based on, for example, displacement of the piston elements **103** during this elapsed time, to determine an internal volume of the SUDS **190.** The internal volume of the SUDS **190** can be converted to length by dividing the internal volume by a known cross-sectional area of the tubing **208.**

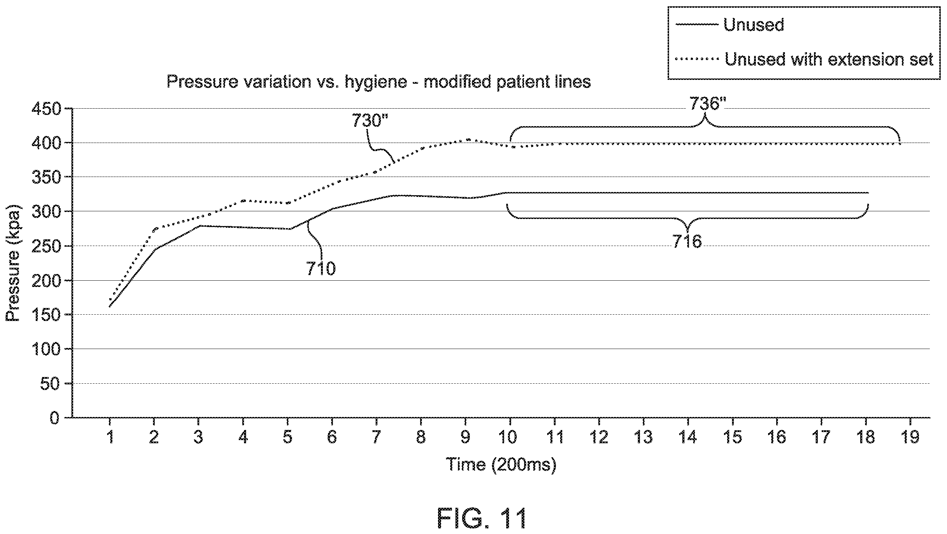

In some aspects or examples, the electronic control unit **900** may determine a presence or absence of an additional fluid path set component connected to the connector **214** of the SUDS **190** based on the distinct pressure profile **730.** The presence of the additional fluid path set component, such as an extension line, may introduce additional characteristics to the distinct pressure profile **730,** such as a third inflection point or an increased steady state pressure. In particular, the additional fluid path set component may introduce an additional restriction to the fluid path, such as an additional one-way check valve or a narrower lumen, that causes a pressure inflection or increased steady state pressure as the priming fluid passes therethrough. **FIG. 11** shows a graph of the first predetermined pressure profile **710** and a modified distinct pressure profile **730"** representative of the SUDS **190** with the additional fluid path set component attached. The electronic control unit **900** may identify the increased pressure of the steady state portion **736"** of the modified distinct pressure profile **730"** relative to the pressure of the steady state portion **716** of the first predetermined pressure profile to determine that the additional fluid path set component is connected to the SUDS **190.**

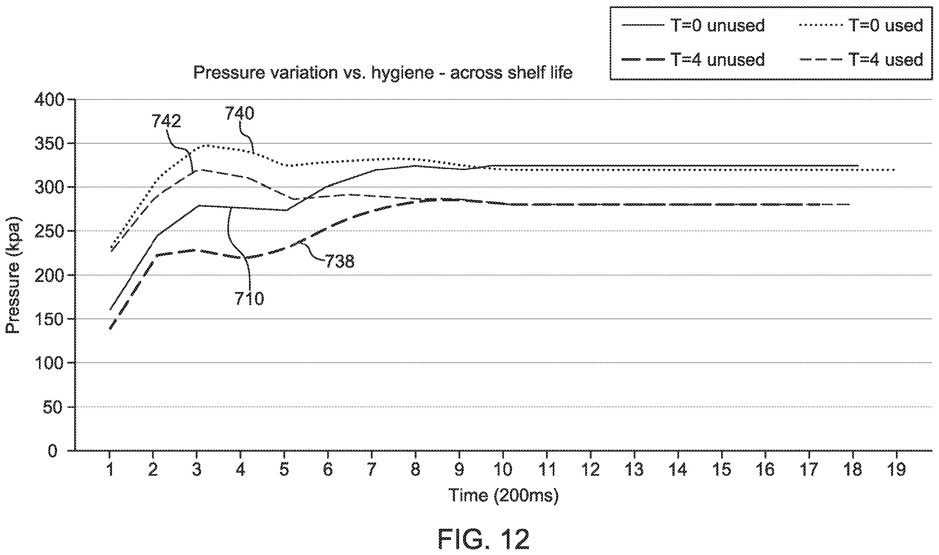

In some aspects or examples, the electronic control unit **900** may account for any differences in pressure profiles depending on an age of the SUDS **190.** **FIG. 12** shows the first predetermined pressure profile **710,** representative of the relatively new or pristine (i.e. not aged) exemplary SUDS, in comparison to an aged second predetermined pressure profile **738** representative of an unused but aged exemplary SUDS. Additionally, a third predetermined pressure profile **740** is representative of a relatively new and previously-used exemplary SUDS, and a fourth predetermined pressure profile **742** is representative of an aged and previously-used exemplary SUDS. Various characteristics of the pressure profiles **710, 738, 740, 742** such as inflection points and steady state portions thereof may be identified, compared, and analyzed by the electronic control unit **900** as generally described herein in steps **808** and **810** of the method **800** to determine the age of the SUDSs represented by the pressure profiles **710, 738, 740, 742.**

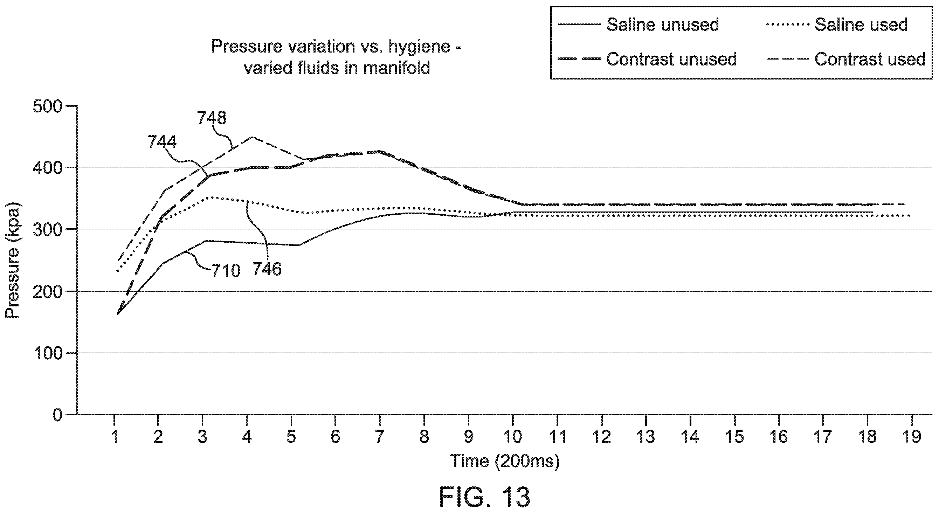

In some aspects or examples, the electronic control unit **900** may account for any differences in pressure profiles depending on what fluid is in the manifold **148** at the start of the priming operation. **FIG. 13** illustrates the first predetermined pressure profile **710** representative of the unused exemplary SUDS coupled with the presence of saline as the fluid in the manifold **148.** Also illustrated are predetermined pressure profiles **744, 746, 748** representative of (i) an unused exemplary SUDS with contrast as the fluid in the manifold **148,** (ii) a previously used exemplary SUDS with saline as the fluid in the manifold **148,** and (iii) a previously used exemplary SUDS with contrast as the fluid in the manifold **148,** respectively. Various characteristics of the predetermined pressure profiles **710, 744, 746, 748,** such as inflection points and steady state portions thereof, may be identified, compared, and analyzed by the electronic control unit **900** as generally described herein in steps **808** and **810** of the method **800.** In this manner, the type of fluid (e.g., saline or contrast) in the manifold **148** at the start of the priming operation can be accounted for during comparison between the distinct pressure profile and the predetermined pressure profile.

In some aspects or examples of the present disclosure, the methods **800, 850,** as well as the other methods and processes described herein, may be implemented in the fluid injector system **100** by a computer program product. The computer program product may include at least one non-transitory computer-readable medium having one or more instructions executable by at least one processor to cause the at least one processor to execute all or part of the method **800.** In some examples or aspects, the at least one non-transitory computer-readable medium and the at least one processor may include or correspond to the memory **908** and processor **904,** respectively, as described above with reference to **FIG. 6****.**

While several examples of fluid injector systems, computer program products, and associated methods are shown in the accompanying drawings and described hereinabove in detail, other examples will be apparent to, and readily made by, those skilled in the art without departing from the scope of the disclosure. For example, it is to be understood that this disclosure contemplates that, to the extent possible, one or more features of any example can be combined with one or more features of any other example. Accordingly, the foregoing description is intended to be illustrative rather than restrictive.

## Claims

1. A fluid injector system (100) configured to perform an injection protocol in connection with a diagnostic imaging procedure, the fluid injector system (100) comprising:
a memory (910) for storing therein a predetermined pressure profile (710), the predetermined pressure profile (710) being representative of pressure expected to be generated within an exemplary administration line (190) by a priming fluid over a course of a priming operation performed on the exemplary administration line (190) during which the priming fluid completely displaces an extant fluid from the exemplary administration line (190);
a control device (900) operatively associated with at least one drive component (103) configured to pressurize and inject at least one fluid through a subject administration line (190) into a patient, the control device (900) including at least one processor (904) programmed or configured to perform an operation comprising:
actuating the at least one drive component (103) to prime the subject administration line with the at least one fluid as the priming fluid;
determining a distinct pressure profile (730) indicative of a measurement of current pressure generated during the priming of the subject administration line with the at least one fluid over the course of the priming operation performed therewith;
comparing the distinct pressure profile (730) to the predetermined pressure profile (710); and
determining, based on a result of the comparison, whether the subject administration line, prior to the priming thereof, contained therein at least one of a liquid as the extant fluid and a gas as the extant fluid.

2. The fluid injector system (100) of claim 1, wherein the exemplary administration line (190) is one of:
(i) an unused administration line and, as a result thereof, the extant fluid therein is the gas and the predetermined pressure profile (710) thereby represents the pressure expected to be generated by the priming fluid during the priming of the unused administration line as the gas therein is completely displaced thereby over the course of the priming operation; and
(ii) a previously used administration line and, as a result thereof, the extant fluid therein is at least partially a liquid and the predetermined pressure profile (710) thereby represents the pressure expected to be generated by the priming fluid during the priming of the previously used administration line as the liquid therein is completely displaced thereby over the course of the priming operation.

3. The fluid injector system (100) of claim 2, wherein the exemplary administration line is the unused administration line (190) and upon the comparison resulting in a correlation within a specified tolerance between the distinct pressure profile (730) and the predetermined pressure profile (710), the at least one processor (904) is configured to determine that the subject administration line contained the gas as the extant fluid.

4. The fluid injector system (100) of claim 3, wherein the operation further comprises:
upon the comparison resulting in the correlation within the specified tolerance between the distinct pressure profile (730) and the predetermined pressure profile (710), generating an alert indicating that the subject administration line had not been used prior to being primed with the at least one fluid during the priming operation.

5. The fluid injection system (100) of claim 3, wherein the operation further comprises:
upon the comparison resulting in the correlation within the specified tolerance between the distinct pressure profile (730) and the predetermined pressure profile (710), permitting performance of the injection protocol.

6. The fluid injector system (100) of claim 3, wherein the subject administration line, like the exemplary administration line (190), comprises at least one check valve (236) precluding fluid flow in a proximal direction; and
wherein the comparison resulting in the correlation within the specified tolerance between the distinct pressure profile (730) and the predetermined pressure profile (710) comprises at least one of:
identifying at least one pressure inflection point in the distinct pressure profile (730) caused by the at least one fluid having passed through the at least one check valve (236) in the subject administration line that correlates to at least one pressure inflection point (722) corresponding thereto in the predetermined pressure profile (710) caused by the priming fluid having passed through the at least one check valve (236) in the exemplary administration line (190);
normalizing the distinct pressure profile (730) about a steady state value thereof, normalizing the predetermined pressure profile (710) about a steady state value thereof, and determining that an area under a curve of the normalized distinct pressure profile correlates to an area under a curve of the predetermined pressure profile (710); and
normalizing the distinct pressure profile (730) about a steady state value thereof, normalizing the predetermined pressure profile (710) about a steady state value thereof, and determining that each point along the distinct pressure profile (730) correlates within a specified tolerance to corresponding points on the predetermined pressure profile (710).

7. The fluid injector system (100) of claim 2, wherein the exemplary administration line (190) is the previously used administration line and upon the comparison resulting in a correlation within a specified tolerance between the distinct pressure profile (730) and the predetermined pressure profile (710), the at least one processor (904) is configured to determine that the subject administration line contained the liquid as the extant fluid.

8. The fluid injector system (100) of claim 7, wherein the operation further comprises:
upon the comparison resulting in the correlation within the specified tolerance between the distinct pressure profile (730) and the predetermined pressure profile (710), generating an alert indicating that the subject administration line had been used prior to being primed with the at least one fluid during the priming operation.

9. The fluid injector system (100) of claim 7, wherein the subject administration line, like the exemplary administration line, comprises at least one check valve (236) precluding fluid flow in a proximal direction; and
wherein the comparison resulting in the correlation within the specified tolerance between the distinct pressure profile (730) and the predetermined pressure profile (710) comprises at least one of:
identifying at least one pressure inflection point (722) in the distinct pressure profile (730) caused by the at least one fluid having passed through the at least one check valve (236) in the subject administration line that correlates to at least one pressure inflection point (722) corresponding thereto in the predetermined pressure profile (710) caused by the priming fluid having passed through the at least one check valve (236) in the exemplary administration line (190);
normalizing the distinct pressure profile (730) about a steady state value thereof, normalizing the predetermined pressure profile (710) about a steady state value thereof, and determining that an area under a curve of the normalized distinct pressure profile (730) correlates to an area under a curve of the predetermined pressure profile (710); and
normalizing the distinct pressure profile (730) about a steady state value thereof, normalizing the predetermined pressure profile (710) about a steady state value thereof, and determining that each point along the distinct pressure profile (730) correlates within a specified tolerance to corresponding points on the predetermined pressure profile (710).

10. The fluid injector system (100) of claim 1, wherein the at least one fluid used in the priming of the subject administration line comprises at least one of (i) a diluent, (ii) a contrast medium and (iii) a mixture of the contrast medium and the diluent.

11. The fluid injector system (100) of claim 1, wherein the determining the distinct pressure profile (730) comprises measuring a motor current of the at least one drive component (103).

12. The fluid injector system (100) of claim 3, wherein the subject administration line, like the exemplary administration line (190), comprises a first check valve (236) precluding fluid flow in a proximal direction and a second check valve (280) precluding flow in the proximal direction, the second check valve (280) located distally of the first check valve (236); and
wherein, during the priming of the subject administration line, the operation further comprises:
identifying a first pressure inflection point (712) in the distinct pressure profile (730) caused by the at least one fluid having passed through the first check valve (236) in the subject administration line; and
identifying at least one of:
a second pressure inflection point (714) in the distinct pressure profile (730) caused by the at least one fluid having passed through the second check valve (280) in the subject administration line; and
a steady state portion of the distinct pressure profile (730) over which the pressure generated within the subject administration line remains substantially constant;
whereupon the subject administration line is determined to be fully primed.

13. The fluid injector system (100) of claim 1, wherein the subject administration line, like the exemplary administration line (190), comprises a single check valve (236) located in a distal end of the subject administration line, the single check valve (236) precluding fluid flow in a proximal direction; and
wherein, during the priming of the subject administration line, the operation further comprises identifying at least one of:
a pressure inflection point (712) in the distinct pressure profile (730) caused by the at least one fluid having passed through the single check valve (236) in the subject administration line; and
a steady state portion of the distinct pressure profile (730) over which the pressure generated within the subject administration line remains substantially constant;
whereupon the subject administration line at a distal end thereof is determined to have a fluid path set component connected thereto.

14. A computer program product for detecting multiple uses of an administration line (190) using a fluid injector system (100) configured to perform an injection protocol in connection with a diagnostic imaging procedure, the computer program product comprising:
non-transitory computer readable media comprising a memory (908) for storing therein a predetermined pressure profile (710), the predetermined pressure profile (710) being representative of pressure expected to be generated within an exemplary administration line (190) by a priming fluid over a course of a priming operation performed on the exemplary administration line (190) during which the priming fluid completely displaces an extant fluid from the exemplary administration line (190);
the non-transitory computer readable media further comprising one or more instructions that, when executed by at least one processor (904), cause the at least one processor (904) to perform an operation comprising:
actuating at least one drive component (103) of the fluid injector system (100) to prime a subject administration line (190) with the priming fluid;
determining a distinct pressure profile indicative of a measurement of current pressure generated during the priming of the subject administration line with the priming fluid over the course of the priming operation performed therewith;
comparing the distinct pressure profile (730) to the predetermined pressure profile (710); and
determining, based on a result of the comparison, whether the subject administration line, prior to the priming thereof, contained therein at least one of a liquid as the extant fluid and a gas as the extant fluid.

15. The computer program product of claim 14, wherein the exemplary administration line (190) is one of:
(i) an unused administration line and, as a result thereof, the extant fluid therein is the gas and the predetermined pressure profile (710) thereby represents the pressure expected to be generated by the priming fluid during the priming of the unused administration line as the gas therein is completely displaced thereby over the course of the priming operation,
wherein, preferably, upon the comparison resulting in a correlation within a specified tolerance between the distinct pressure profile (730) and the predetermined pressure profile (710), the one or more instructions, when executed by the at least one processor (904), cause the at least one processor (904) to perform a further operation comprising:
determining that the subject administration line contained the gas as the extant fluid,
generating an alert indicating that the subject administration line had not been used prior to being primed with the priming fluid during the priming operation, and
permitting performance of the injection protocol; and
(ii) a previously used administration line and, as a result thereof, the extant fluid therein is at least partially a liquid and the predetermined pressure profile (710) thereby represents the pressure expected to be generated by the priming fluid during the priming of the previously used administration line as the liquid therein is completely displaced thereby over the course of the priming operation.

## Patentansprüche

1. Ein Fluidinjektionssystem (100), das so konfiguriert ist, dass es ein Injektionsprotokoll in Verbindung mit einem diagnostischen Bildgebungsverfahren durchführt, wobei das Fluidinjektionssystem (100) Folgendes umfasst:
einen Speicher (910), um darin ein vorbestimmtes Druckprofil (710) zu speichern, wobei das vorbestimmte Druckprofil (710) repräsentativ für den Druck ist, der in einer beispielhaften Leitung zur Fluidzufuhr (190) durch ein Entlüftungsfluid im Verlauf eines Entlüftungsvorgangs erzeugt werden soll, der an der beispielhaften Leitung zur Fluidzufuhr (190) durchgeführt wird, während dessen das Entlüftungsfluid ein vorhandenes Fluid vollständig aus der beispielhaften Leitung zur Fluidzufuhr (190) verdrängt;
eine Steuervorrichtung (900), die funktionsfähig mit mindestens einer Antriebskomponente (103) verbunden ist, die so konfiguriert ist, dass sie mindestens ein Fluid mit Druck beaufschlagt und durch eine Patientenleitung zur Fluidzufuhr (190) in einen Patienten injiziert, wobei die Steuervorrichtung (900) mindestens einen Prozessor (904) enthält, der so programmiert oder konfiguriert ist, dass er einen Vorgang durchführt, der Folgendes umfasst:
Betätigen der mindestens einen Antriebskomponente (103), um die Patientenleitung zur Fluidzufuhr mit dem mindestens einen Fluid als Entlüftungsfluid zu entlüften;
Bestimmen eines bestimmten Druckprofils (730), das auf eine Messung des aktuellen Drucks hinweist, der während des Entlüftens der Patientenleitung zur Fluidzufuhr mit dem mindestens einen Fluid im Verlauf des damit durchgeführten Entlüftungsvorgangs erzeugt wird;
Vergleichen des bestimmten Druckprofils (730) mit dem vorbestimmten Druckprofil (710); und
Bestimmen, basierend auf einem Ergebnis des Vergleichs, ob die Patientenleitung zur Fluidzufuhr vor dem Entlüften mindestens eine Flüssigkeit als das vorhandene Fluid oder ein Gas als das vorhandene Fluid enthielt.

2. Das Fluidinjektionssystem (100) nach Anspruch 1, wobei die beispielhafte Leitung zur Fluidzufuhr (190) eine der folgenden ist:
(i) eine unbenutzte Leitung zur Fluidzufuhr ist und, als Folge davon, das darin vorhandene Fluid das Gas ist und das vorbestimmte Druckprofil (710) dadurch den Druck repräsentiert, der voraussichtlich durch das Entlüftungsfluid während des Entlüftens der unbenutzten Leitung zur Fluidzufuhr erzeugt wird, wenn das darin befindliche Gas im Verlauf des Entlüftungsvorgangs vollständig verdrängt wird; und
(ii) eine zuvor benutzte Leitung zur Fluidzufuhr, wobei das darin befindliche Fluid zumindest teilweise eine Flüssigkeit ist und das vorbestimmte Druckprofil (710) dadurch den Druck repräsentiert, der von dem Entlüftungsfluid während des Entlüftens der zuvor benutzten Leitung zur Fluidzufuhr voraussichtlich erzeugt wird, wenn die darin befindliche Flüssigkeit im Verlauf des Entlüftungsvorgangs vollständig verdrängt wird.

3. Das Fluidinjektionssystem (100) nach Anspruch 2, wobei die beispielhafte Leitung zur Fluidzufuhr die unbenutzte Leitung zur Fluidzufuhr (190) ist und der mindestens eine Prozessor (904) so konfiguriert ist, dass er bei dem Vergleich, der zu einer Korrelation innerhalb einer spezifizierten Toleranz zwischen dem bestimmten Druckprofil (730) und dem vorbestimmten Druckprofil (710) führt, feststellt, dass die Patientenleitung zur Fluidzufuhr das Gas als das vorhandene Fluid enthält.

4. Das Fluidinjektionssystem (100) nach Anspruch 3, wobei der Vorgang ferner Folgendes umfasst:
auf den Vergleich hin, der zu einer Korrelation innerhalb der spezifizierten Toleranz zwischen dem bestimmten Druckprofil (730) und dem vorbestimmten Druckprofil (710) führt, das Erzeugen eines Alarms, der anzeigt, dass die Patientenleitung zur Fluidzufuhr nicht benutzt wurde, bevor sie während des Entlüftungsvorgangs mit dem mindestens einen Fluid beaufschlagt wurde.

5. Das Fluidinjektionssystem (100) nach Anspruch 3, wobei der Vorgang ferner umfasst:
auf den Vergleich hin, der die Korrelation innerhalb der spezifizierten Toleranz zwischen dem bestimmten Druckprofil (730) und dem vorbestimmten Druckprofil (710) ergibt, die Durchführung des Injektionsprotokolls ermöglicht.

6. Das Fluidinjektionssystem (100) nach Anspruch 3, wobei die Patientenleitung zur Fluidzufuhr, wie die beispielhafte Leitung zur Fluidzufuhr (190), mindestens ein Rückschlagventil (236) umfasst, das den Fluidfluss in einer proximalen Richtung ausschließt; und
wobei der Vergleich, der zu der Korrelation innerhalb der spezifizierten Toleranz zwischen dem bestimmten Druckprofil (730) und dem vorbestimmten Druckprofil (710) führt, mindestens eines von Folgendem umfasst:
Identifizieren mindestens eines Druckwendepunkts in dem bestimmten Druckprofil (730), der dadurch bewirkt wird, dass das mindestens eine Fluid das mindestens eine Rückschlagventil (236) in der Patientenleitung zur Fluidzufuhr passiert hat, und der mit mindestens einem diesem entsprechenden Druckwendepunkt (722) in dem vorbestimmten Druckprofil (710) korreliert, der dadurch bewirkt wird, dass das Entlüftungsfluid das mindestens eine Rückschlagventil (236) in der beispielhaften Leitung zur Fluidzufuhr (190) passiert hat;
Normalisieren des bestimmten Druckprofils (730) um einen stationären Wert davon, Normalisieren des vorbestimmten Druckprofils (710) um einen stationären Wert davon, und Bestimmen, dass eine Fläche unter einer Kurve des normalisierten bestimmten Druckprofils mit einer Fläche unter einer Kurve des vorbestimmten Druckprofils (710) korreliert; und
Normalisieren des bestimmten Druckprofils (730) um einen stationären Wert davon, Normalisieren des vorbestimmten Druckprofils (710) um einen stationären Wert davon und Bestimmen, dass jeder Punkt entlang des bestimmten Druckprofils (730) innerhalb einer spezifizierten Toleranz mit entsprechenden Punkten auf dem vorbestimmten Druckprofil (710) korreliert.

7. Das Fluidinjektionssystem (100) nach Anspruch 2, wobei die beispielhafte Leitung zur Fluidzufuhr (190) die zuvor verwendete Leitung zur Fluidzufuhr ist und der mindestens eine Prozessor (904) so konfiguriert ist, dass er bei dem Vergleich, der zu einer Korrelation innerhalb einer spezifizierten Toleranz zwischen dem bestimmten Druckprofil (730) und dem vorbestimmten Druckprofil (710) führt, feststellt, dass die Patientenleitung zur Fluidzufuhr die Flüssigkeit als das vorhandene Fluid enthält.

8. Das Fluidinjektionssystem (100) nach Anspruch 7, wobei der Vorgang ferner Folgendes umfasst:
auf den Vergleich hin, der zu einer Korrelation innerhalb der spezifizierten Toleranz zwischen dem bestimmten Druckprofil (730) und dem vorbestimmten Druckprofil (710) führt, das Erzeugen eines Alarms, der anzeigt, dass die Patientenleitung zur Fluidzufuhr verwendet wurde, bevor sie während des Entlüftens mit dem mindestens einen Fluid beaufschlagt wurde.

9. Das Fluidinjektionssystem (100) nach Anspruch 7, wobei die Patientenleitung zur Fluidzufuhr, wie die beispielhafte Leitung zur Fluidzufuhr, mindestens ein Rückschlagventil (236) umfasst, das den Fluidfluss in einer proximalen Richtung ausschließt; und
wobei der Vergleich, der zu der Korrelation innerhalb der spezifizierten Toleranz zwischen dem bestimmten Druckprofil (730) und dem vorbestimmten Druckprofil (710) führt, mindestens eines von Folgendem umfasst:
Identifizieren mindestens eines Druckwendepunkts (722) in dem bestimmten Druckprofil (730), der dadurch bewirkt wird, dass das mindestens eine Fluid das mindestens eine Rückschlagventil (236) in der Patientenleitung zur Fluidzufuhr passiert hat, und der mit mindestens einem diesem entsprechenden Druckwendepunkt (722) in dem vorbestimmten Druckprofil (710) korreliert, der dadurch bewirkt wird, dass das Entlüftungsfluid das mindestens eine Rückschlagventil (236) in der beispielhaften Leitung zur Fluidzufuhr (190) passiert hat;
Normalisieren des bestimmten Druckprofils (730) um einen stationären Wert davon, Normalisieren des vorbestimmten Druckprofils (710) um einen stationären Wert davon, und Bestimmen, dass eine Fläche unter einer Kurve des normalisierten bestimmten Druckprofils (730) mit einer Fläche unter einer Kurve des vorbestimmten Druckprofils (710) korreliert; und
Normalisieren des bestimmten Druckprofils (730) um einen stationären Wert davon, Normalisieren des vorbestimmten Druckprofils (710) um einen stationären Wert davon und Bestimmen, dass jeder Punkt entlang des bestimmten Druckprofils (730) innerhalb einer spezifizierten Toleranz mit entsprechenden Punkten auf dem vorbestimmten Druckprofil (710) korreliert.

10. Das Fluidinjektionssystem (100) nach Anspruch 1, wobei das mindestens eine beim Entlüften der Patientenleitung zur Fluidzufuhr verwendete Fluid mindestens eines der folgenden umfasst
(i) ein Verdünnungsmittel,
(ii) ein Kontrastmittel und
(iii) eine Mischung aus dem Kontrastmittel und dem Verdünnungsmittel.

11. Das Fluidinjektionssystem (100) nach Anspruch 1, wobei das Bestimmen des bestimmten Druckprofils (730) die Messung eines Motorstroms der mindestens einen Antriebskomponente (103) umfasst.

12. Das Fluidinjektionssystem (100) nach Anspruch 3, wobei die Patientenleitung zur Fluidzufuhr, wie die beispielhafte Leitung zur Fluidzufuhr (190), ein erstes Rückschlagventil (236) umfasst, das den Fluidfluss in einer proximalen Richtung ausschließt, und ein zweites Rückschlagventil (280), das den Fluss in der proximalen Richtung ausschließt, wobei das zweite Rückschlagventil (280) distal von dem ersten Rückschlagventil (236) angeordnet ist; und
wobei der Vorgang während des Entlüftens der Patientenleitung zur Fluidzufuhr ferner folgendes umfasst:
Identifizieren eines ersten Druckwendepunkts (712) in dem bestimmten Druckprofil (730), der dadurch bewirkt wird, dass das mindestens eine Fluid das erste Rückschlagventil (236) in der Patientenleitung zur Fluidzufuhr passiert hat; und
Identifizieren von mindestens einem von:
einem zweiten Druckwendepunkt (714) in dem bestimmten Druckprofil (730), der dadurch bewirkt wird, dass das mindestens eine Fluid das zweite Rückschlagventil (280) in der Patientenleitung zur Fluidzufuhr passiert hat;
und
einen stationären Abschnitt des bestimmten Druckprofils (730), über den der in der Patientenleitung zur Fluidzufuhr erzeugte Druck im Wesentlichen konstant bleibt;
woraufhin festgestellt wird, dass die Patientenleitung zur Fluidzufuhr vollständig entlüftet ist.

13. Das Fluidinjektionssystem (100) nach Anspruch 1, wobei die Patientenleitung zur Fluidzufuhr, wie die beispielhafte Leitung zur Fluidzufuhr (190), ein einzelnes Rückschlagventil (236) umfasst, das in einem distalen Ende der Patientenleitung zur Fluidzufuhr angeordnet ist, wobei das einzelne Rückschlagventil (236) den Flüssigkeitsfluss in einer proximalen Richtung ausschließt; und
wobei der Vorgang während des Entlüftens der Patientenleitung zur Fluidzufuhr ferner das Identifizieren von mindestens einem der folgenden Punkte umfasst:
einem Druckwendepunkt (712) in dem bestimmten Druckprofil (730), der dadurch bewirkt wird, dass das mindestens eine Fluid das einzelne Rückschlagventil (236) in der Patientenleitung zur Fluidzufuhr passiert hat; und
einen stationären Abschnitt des bestimmten Druckprofils (730), über den der in der Patientenleitung zur Fluidzufuhr erzeugte Druck im Wesentlichen konstant bleibt;
woraufhin festgestellt wird, dass an einem distalen Ende der Patientenleitung zur Fluidzufuhr eine Fluidpfadkomponente angeschlossen ist.

14. Ein Computerprogrammprodukt zum Erkennen mehrfacher Verwendungen einer Leitung zur Fluidzufuhr (190) unter Verwendung eines Fluidinjektionssystems (100), das so konfiguriert ist, dass es ein Injektionsprotokoll in Verbindung mit einem diagnostischen Bildgebungsverfahren durchführt, wobei das Computerprogrammprodukt Folgendes umfasst:
nicht transitorische computerlesbare Medien, umfassend einen Speicher (908), um darin ein vorbestimmtes Druckprofil (710) zu speichern, wobei das vorbestimmte Druckprofil (710) repräsentativ für den Druck ist, von dem erwartet wird, dass er innerhalb einer beispielhaften Leitung zur Fluidzufuhr (190) durch ein Entlüftungsfluid über einen Verlauf eines Entlüftungsvorgangs erzeugt wird, der an der beispielhaften Leitung zur Fluidzufuhr (190) durchgeführt wird, während dessen das Entlüftungsfluid ein vorhandenes Fluid vollständig aus der beispielhaften Leitung zur Fluidzufuhr (190) verdrängt;
wobei das nicht-transitorische computerlesbare Medium ferner einen oder mehrere Befehle umfasst, die, wenn sie von mindestens einem Prozessor (904) ausgeführt werden, den mindestens einen Prozessor (904) veranlassen, einen Vorgang durchzuführen, die Folgendes umfasst:
Betätigen mindestens einer Antriebskomponente (103) des Fluidinjektionssystems (100), um eine Patientenleitung zur Fluidzufuhr (190) mit dem Entlüftungsfluid zu entlüften;
Bestimmen eines bestimmten Druckprofils, das auf eine Messung des aktuellen Drucks hinweist, der während des Entlüftens der Patientenleitung zur Fluidzufuhr mit dem Entlüftungsfluid im Verlauf des damit durchgeführten Entlüftungsvorgangs erzeugt wird;
Vergleichen des bestimmten Druckprofils (730) mit dem vorbestimmten Druckprofil (710); und
Bestimmen, basierend auf einem Ergebnis des Vergleichs, ob die Patientenleitung zur Fluidzufuhr vor dem Entlüften derselben mindestens eine Flüssigkeit als das vorhandene Fluid oder ein Gas als das vorhandene Fluid enthielt.

15. Das Computerprogramm nach Anspruch 14, wobei die beispielhafte Leitung zur Fluidzufuhr (190) eine der folgenden ist:
(i) eine unbenutzte Leitung zur Fluidzufuhr ist und, als Ergebnis davon, das vorhandene Fluid darin das Gas ist und das vorbestimmte Druckprofil (710) dadurch den Druck repräsentiert, von dem erwartet wird, dass er durch das Entlüftungsfluid während des Entlüftens der unbenutzten Leitung zur Fluidzufuhr erzeugt wird, wenn das Gas darin im Verlauf des Entlüftungsvorgangs vollständig verdrängt wird,
wobei, bevorzugt, nach dem Vergleich, der zu einer Korrelation innerhalb einer spezifizierten Toleranz zwischen dem eindeutigen Druckprofil (730) und dem vorbestimmten Druckprofil (710) führt, die eine oder die mehreren Anweisungen, wenn sie von dem mindestens einen Prozessor (904) ausgeführt werden, den mindestens einen Prozessor (904) veranlassen, einen weiteren Vorgang durchzuführen, umfassend:
Feststellen, dass die Patientenleitung zur Fluidzufuhr das Gas als bestehende Flüssigkeit enthielt,
Erzeugen eines Alarms, der anzeigt, dass die Patientenleitung zur Fluidzufuhr nicht benutzt wurde, bevor sie während des Entlüftungsvorgangs mit dem Entlüftungsfluid entlüftet wurde, und
Erlauben der Durchführung des Injektionsprotokolls; und
(ii) eine zuvor benutzte Leitung zur Fluidzufuhr und, als Ergebnis davon, ist das vorhandene Fluid darin zumindest teilweise eine Flüssigkeit, und das vorbestimmte Druckprofil (710) repräsentiert dadurch den Druck, von dem erwartet wird, dass er durch das Entlüftungsfluid während des Entlüftens der zuvor benutzten Leitung zur Fluidzufuhr erzeugt wird, wenn die Flüssigkeit darin im Verlauf des Entlüftungsvorgangs vollständig verdrängt wird.

## Revendications

1. Système d'injection de fluide (100) configuré pour exécuter un protocole d'injection en relation avec une procédure d'imagerie diagnostique, le système d'injection de fluide (100) comprenant :
une mémoire (910) pour y stocker un profil de pression prédéterminé (710), le profil de pression prédéterminé (710) étant représentatif de la pression qui devrait être générée dans une ligne d'administration exemplaire (190) par un fluide d'amorçage au cours d'une opération d'amorçage effectuée sur la ligne d'administration exemplaire (190) au cours de laquelle le fluide d'amorçage déplace complètement un fluide existant de la ligne d'administration exemplaire (190) ;
un dispositif de commande (900) associé de manière opérationnelle à au moins un composant d'entraînement (103) configuré pour pressuriser et injecter au moins un fluide à travers une ligne d'administration du sujet (190) dans un patient, le dispositif de commande (900) comprenant au moins un processeur (904) programmé ou configuré pour effectuer une opération comprenant :
actionner au moins un composant d'entraînement (103) pour amorcer la ligne d'administration du sujet avec au moins un fluide comme fluide d'amorçage ;
déterminer un profil de pression distinct (730) indiquant une mesure de la pression actuelle générée pendant l'amorçage de la ligne d'administration du sujet avec au moins un fluide au cours de l'opération d'amorçage effectuée avec celle-ci ;
comparer le profil de pression distinct (730) au profil de pression prédéterminé (710) ; et
déterminer, sur la base du résultat de la comparaison, si la ligne d'administration en question, avant son amorçage, contenait au moins l'un des deux fluides suivants :
un liquide en tant que fluide extant et un gaz en tant que fluide extant.

2. Le système d'injection de fluide (100) de la revendication 1, en ce que la ligne d'administration exemplaire (190) est l'une des suivantes :
(i) une conduite d'administration inutilisée et, par conséquent, le fluide existant à l'intérieur est le gaz et le profil de pression prédéterminé (710) représente ainsi la pression qui devrait être générée par le fluide d'amorçage pendant l'amorçage de la conduite d'administration inutilisée à mesure que le gaz à l'intérieur est complètement déplacé au cours de l'opération d'amorçage ; et
(ii) une ligne d'administration précédemment utilisée et, en conséquence, le fluide existant dans cette ligne est au moins partiellement un liquide et le profil de pression prédéterminé (710) représente ainsi la pression qui devrait être générée par le fluide d'amorçage pendant l'amorçage de la ligne d'administration précédemment utilisée lorsque le liquide qu'elle contient est complètement déplacé au cours de l'opération d'amorçage.

3. Le système d'injection de fluide (100) de la revendication 2, dans lequel la ligne d'administration exemplaire est la ligne d'administration non utilisée (190) et sur la base de la comparaison résultant en une corrélation dans une tolérance spécifiée entre le profil de pression distinct (730) et le profil de pression prédéterminé (710), l'au moins un processeur (904) est configuré pour déterminer que la ligne d'administration sujette contenait le gaz en tant que fluide extant.

4. Le système d'injection de fluide (100) de la revendication 3, selon laquelle l'opération comprend en outre :
lors de la comparaison aboutissant à la corrélation dans la tolérance spécifiée entre le profil de pression distinct (730) et le profil de pression prédéterminé (710), générer une alerte indiquant que la ligne d'administration concernée n'avait pas été utilisée antérieurement avant d'être amorcée avec l'au moins un fluide au cours de l'opération d'amorçage.

5. Système d'injection de fluide (100) selon la revendication 3, car l'opération comprend en outre :
lors de la comparaison résultant de la corrélation dans la tolérance spécifiée entre le profil de pression distinct (730) et le profil de pression prédéterminé (710), permettre l'exécution du protocole d'injection.

6. Le système d'injection de fluide (100) de la revendication 3, car la ligne d'administration concernée, comme la ligne d'administration exemplaire (190), comprend au moins un clapet anti-retour (236) empêchant l'écoulement du fluide dans une direction proximale ; et
**Caractérisée en ce que** la comparaison aboutissant à la corrélation dans la tolérance spécifiée entre le profil de pression distinct (730) et le profil de pression prédéterminé (710) comprend au moins l'un des éléments suivants :
l'identification d'au moins un point d'inflexion de pression dans le profil de pression distinct (730) causé par l'au moins un fluide ayant traversé l'au moins un clapet anti-retour (236) dans la conduite d'administration concernée qui est en corrélation avec au moins un point d'inflexion de pression (722) correspondant dans le profil de pression prédéterminé (710) causé par le fluide d'amorçage ayant traversé l'au moins un clapet anti-retour (236) dans la conduite d'administration exemplaire (190) ;
normaliser le profil de pression distinct (730) autour d'une valeur d'état stable, normaliser le profil de pression prédéterminé (710) autour d'une valeur d'état stable, et déterminer qu'une aire sous une courbe du profil de pression distinct normalisé est en corrélation avec une aire sous une courbe du profil de pression prédéterminé (710) ; et
normaliser le profil de pression distinct (730) autour d'une valeur de régime permanent, normaliser le profil de pression prédéterminé (710) autour d'une valeur de régime permanent, et déterminer que chaque point du profil de pression distinct (730) est en corrélation, dans une tolérance spécifiée, avec les points correspondants du profil de pression prédéterminé (710).

7. Le système d'injection de fluide (100) de la revendication 2, dans lequel la ligne d'administration exemplaire (190) est la ligne d'administration précédemment utilisée et, sur la base de la comparaison résultant en une corrélation dans une tolérance spécifiée entre le profil de pression distinct (730) et le profil de pression prédéterminé (710), l'au moins un processeur (904) est configuré pour déterminer que la ligne d'administration en question contenait le liquide en tant que fluide extant.

8. Le système d'injection de fluide (100) de la revendication 7, selon laquelle l'opération comprend en outre :
lors de la comparaison aboutissant à la corrélation dans la tolérance spécifiée entre le profil de pression distinct (730) et le profil de pression prédéterminé (710), la génération d'une alerte indiquant que la ligne d'administration concernée avait été utilisée antérieurement à l'amorçage avec l'au moins un fluide au cours de l'opération d'amorçage.

9. Le système d'injection de fluide (100) de la revendication 7, dans lequel la ligne d'administration concernée, comme la ligne d'administration exemplaire, comprend au moins un clapet anti-retour (236) empêchant l'écoulement du fluide dans une direction proximale ; et
**Caractérisée en ce que** la comparaison aboutissant à la corrélation dans la tolérance spécifiée entre le profil de pression distinct (730) et le profil de pression prédéterminé (710) comprend au moins l'un des éléments suivants :
l'identification d'au moins un point d'inflexion de pression (722) dans le profil de pression distinct (730) causé par l'au moins un fluide ayant traversé l'au moins un clapet anti-retour (236) dans la ligne d'administration concernée qui est en corrélation avec au moins un point d'inflexion de pression (722) correspondant dans le profil de pression prédéterminé (710) causé par le fluide d'amorçage ayant traversé l'au moins un clapet anti-retour (236) dans la ligne d'administration exemplaire (190) ;
normaliser le profil de pression distinct (730) autour d'une valeur d'état stable, normaliser le profil de pression prédéterminé (710) autour d'une valeur d'état stable, et déterminer qu'une aire sous une courbe du profil de pression distinct normalisé (730) est en corrélation avec une aire sous une courbe du profil de pression prédéterminé (710) ; et
normaliser le profil de pression distinct (730) autour d'une valeur de régime permanent, normaliser le profil de pression prédéterminé (710) autour d'une valeur de régime permanent, et déterminer que chaque point du profil de pression distinct (730) est en corrélation, dans une tolérance spécifiée, avec les points correspondants du profil de pression prédéterminé (710).

10. Le système d'injection de fluide (100) de la revendication 1, dans lequel au moins un fluide utilisé dans l'amorçage de la ligne d'administration du sujet comprend au moins l'un des éléments suivants
(i) un diluant
(ii) un agent de contraste et
(iii) un mélange de produit de contraste et de diluant.

11. Le système d'injection de fluide (100) de la revendication 1, selon laquelle la détermination du profil de pression distinct (730) comprend la mesure d'un courant de moteur du au moins un composant d'entraînement (103).

12. Système d'injection de fluide (100) de la revendication 3, dans lequel la ligne d'administration du sujet, comme la ligne d'administration exemplaire (190), comprend un premier clapet anti-retour (236) empêchant l'écoulement du fluide dans une direction proximale et un second clapet anti-retour (280) empêchant l'écoulement dans la direction proximale, le second clapet anti-retour (280) étant situé distalement par rapport au premier clapet anti-retour (236) ; et
**Caractérisée en ce que**, pendant l'amorçage de la ligne d'administration du sujet, l'opération comprend en outre :
l'identification d'un premier point d'inflexion de pression (712) dans le profil de pression distinct (730) causé par le fait qu'au moins un fluide a traversé le premier clapet anti-retour (236) dans la ligne d'administration du sujet ; et
identifier au moins l'un des éléments suivants
un deuxième point d'inflexion de la pression (714) dans le profil de pression distinct (730) causé par le fait qu'au moins un fluide a traversé le deuxième clapet anti-retour (280) dans la ligne d'administration du sujet ; et
une portion de régime permanent du profil de pression distinct (730) au cours de laquelle la pression générée à l'intérieur de la conduite d'administration en question reste notamment constante ;
après quoi la ligne d'administration du sujet est déterminée comme étant complètement amorcée.

13. Le système d'injection de fluide (100) de la revendication 1, dans lequel la ligne d'administration du sujet, comme la ligne d'administration exemplaire (190), comprend un clapet anti-retour unique (236) situé dans une extrémité distale de la ligne d'administration du sujet, le clapet anti-retour unique (236) empêchant l'écoulement du fluide dans une direction proximale ; et
**Caractérisée en ce que**, pendant l'amorçage de la ligne d'administration du sujet, l'opération comprend en outre l'identification d'au moins un parmi :
un point d'inflexion de pression (712) dans le profil de pression distinct (730) causé par le fait qu'au moins un fluide a traversé le clapet anti-retour unique (236) dans la conduite d'administration du sujet ; et
une portion de régime permanent du profil de pression distinct (730) au cours de laquelle la pression générée dans la canalisation d'administration en question reste notamment substantielle ;
après quoi il est déterminé que la ligne d'administration du sujet, à son extrémité distale, est reliée à un composant de l'ensemble du trajet du fluide.

14. Produit de programme informatique permettant de détecter les utilisations multiples d'une ligne d'administration (190) à l'aide d'un système d'injecteur de fluide (100) configuré pour exécuter un protocole d'injection en relation avec une procédure d'imagerie diagnostique, le produit de programme informatique comprenant :
un support lisible par ordinateur non transitoire comprenant une mémoire (908) pour y stocker un profil de pression prédéterminé (710), le profil de pression prédéterminé (710) étant représentatif de la pression censée être générée à l'intérieur d'une ligne d'administration exemplaire (190) par un fluide d'amorçage au cours d'une opération d'amorçage effectuée sur la ligne d'administration exemplaire (190) au cours de laquelle le fluide d'amorçage déplace complètement un fluide existant de la ligne d'administration exemplaire (190) ;
Le support lisible par ordinateur non transitoire comprend en outre une ou plusieurs instructions qui, lorsqu'elles sont exécutées par au moins un processeur (904), amènent l'au moins un processeur (904) à effectuer une opération comprenant :
actionner au moins un composant d'entraînement (103) du système d'injection de fluide (100) pour amorcer une ligne d'administration du sujet (190) avec le fluide d'amorçage ;
déterminer un profil de pression distinct indiquant une mesure de la pression actuelle générée pendant l'amorçage de la ligne d'administration du sujet avec le fluide d'amorçage au cours de l'opération d'amorçage effectuée avec celui-ci ;
comparer le profil de pression distinct (730) au profil de pression prédéterminé (710) ; et
déterminer, sur la base du résultat de la comparaison, si la conduite d'administration concernée, avant son amorçage, contenait au moins l'un des deux fluides suivants : un liquide en tant que fluide extant et un gaz en tant que fluide extant.

15. Le programme ordinateur de la revendication 14, dans lequel la ligne d'administration exemplaire (190) est l'une des suivantes :
(i) une conduite d'administration inutilisée et, par conséquent, le fluide existant à l'intérieur est le gaz et le profil de pression prédéterminé (710) représente ainsi la pression qui devrait être générée par le fluide d'amorçage pendant l'amorçage de la conduite d'administration inutilisée lorsque le gaz à l'intérieur est complètement déplacé au cours de l'opération d'amorçage,
caratérisée en ce que, de préférence, sur la comparaison résultant en une corrélation dans une tolérance spécifiée entre le profil de pression distinct (730) et le profil de pression prédéterminé (710), l'une ou plusieurs instructions, lorsqu'elles sont exécutées par l'au moins un processeur (904), amènent l'au moins un processeur (904) à effectuer une opération supplémentaire comprenant :
déterminer que la ligne d'administration du sujet contenait le gaz en tant que fluide extant,
générer une alerte indiquant que la ligne d'administration du sujet n'a pas été utilisée antérieurement à l'amorçage avec le fluide d'amorçage au cours de l'opération d'amorçage, et
autoriser l'exécution du protocole d'injection ; et
(ii) une ligne d'administration précédemment utilisée et, par conséquent, le fluide restant est au moins partiellement un liquide et le profil de pression prédéterminé (710) représente ainsi la pression qui devrait être générée par le fluide d'amorçage pendant l'amorçage de la ligne d'administration précédemment utilisée lorsque le liquide est complètement déplacé au cours de l'opération d'amorçage.
